(19) **Europäisches Patentamt / European Patent Office / Office européen des brevets**

(11) **EP 3 973 503 B1**

(12) **FASCICULE DE BREVET EUROPEEN**

(45) Date de publication et mention de la délivrance du brevet:
**06.08.2025 Bulletin 2025/32**

(21) Numéro de dépôt: **20725200.8**

(22) Date de dépôt: **18.05.2020**

(51) Classification Internationale des Brevets (IPC):
**G06T 7/00** (2017.01)

(52) Classification Coopérative des Brevets (CPC):
**G06T 7/0012**

(86) Numéro de dépôt international:
**PCT/EP2020/063797**

(87) Numéro de publication internationale:
**WO 2020/234232 (26.11.2020 Gazette 2020/48)**

(54) **PROCEDE, DISPOSITIF ET SUPPORT LISIBLE PAR ORDINATEUR POUR DETECTER AUTOMATIQUEMENT UNE STENOSE CORONARIENNE HEMODYNAMIQUEMENT SIGNIFICATIVE**

VERFAHREN, VORRICHTUNG UND COMPUTERLESBARES MEDIUM ZUR AUTOMATISCHEN ERKENNUNG EINER HÄMODYNAMISCH SIGNIFIKANTEN KORONARSTENOSE

METHOD, DEVICE AND COMPUTER-READABLE MEDIUM FOR AUTOMATICALLY DETECTING A HAEMODYNAMICALLY SIGNIFICANT CORONARY STENOSIS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.05.2019 FR 1905415**

(43) Date de publication de la demande:
**30.03.2022 Bulletin 2022/13**

(73) Titulaire: **SPIMED-AI**
**75014 Paris (FR)**

(72) Inventeur: **Paul, Jean-Francois**
**92340 Bourg-la-Reine (FR)**

(74) Mandataire: **Icosa**
**83 avenue Denfert-Rochereau**
**75014 Paris (FR)**

(56) Documents cités:
**US-A1- 2015 112 182**

• **YOUNGTAEK HONG: "Novel Methodology for Coronary Artery Disease Evaluation: From a New Imaging Technique to Deep Learning Based Quantification", 1 June 2018 (2018-06-01), The Graduate School, Yonsei University, XP055660869, Retrieved from the Internet <URL:https://ir.ymlib.yonsei.ac.kr/bitstream/22282913/166394/1/T014867.pdf> [retrieved on 20200122]**
• **VAN ROSENDAEL ALEXANDER R ET AL: "Maximization of the usage of coronary CTA derived plaque information using a machine learning based algorithm to improve risk stratification; insights from the CONFIRM registry", JOURNAL OF CARDIOVASCULAR COMPUTED TOMOGRAPHY, ELSEVIER, AMSTERDAM, NL, vol. 12, no. 3, 30 April 2018 (2018-04-30), pages 204 - 209, XP085408791, ISSN: 1934-5925, DOI: 10.1016/J.JCCT.2018.04.011**
• **MAJD ZREIK ET AL: "A Recurrent CNN for Automatic Detection and Classification of Coronary Artery Plaque and Stenosis in Coronary CT Angiography", ARXIV.ORG, CORNELL UNIVERSITY LIBRARY, 201 OLIN LIBRARY CORNELL UNIVERSITY ITHACA, NY 14853, 12 April 2018 (2018-04-12), XP081431870, DOI: 10.1109/TMI.2018.2883807**

- NAKANISHI RINE ET AL: "Automated estimation of image quality for coronary computed tomographic angiography using machine learning", EUROPEAN RADIOLOGY, SPRINGER INTERNATIONAL, BERLIN, DE, vol. 28, no. 9, 23 March 2018 (2018-03-23), pages 4018 - 4026, XP036562550, ISSN: 0938-7994, [retrieved on 20180323], DOI: 10.1007/S00330-018-5348-8
- CANO-ESPINOSA CARLOS ET AL: "Automated Agatston score computation in non-ECG gated CT scans using deep learning", PROGRESS IN BIOMEDICAL OPTICS AND IMAGING, SPIE - INTERNATIONAL SOCIETY FOR OPTICAL ENGINEERING, BELLINGHAM, WA, US, vol. 10574, 2 March 2018 (2018-03-02), pages 105742K - 105742K, XP060103149, ISSN: 1605-7422, ISBN: 978-1-5106-0027-0, DOI: 10.1117/12.2293681
- KOLOSSVÁRY MÁRTON ET AL: "Advanced atherosclerosis imaging by CT: Radiomics, machine learning and deep learning", JOURNAL OF CARDIOVASCULAR COMPUTED TOMOGRAPHY, ELSEVIER, AMSTERDAM, NL, vol. 13, no. 5, 21 April 2019 (2019-04-21), pages 274 - 280, XP085967635, ISSN: 1934-5925, [retrieved on 20190421], DOI: 10.1016/J.JCCT.2019.04.007

## Description

La présente invention concerne un procédé implémenté par ordinateur pour déterminer automatiquement la présence d'une sténose coronarienne hémodynamiquement significative en prédisant la valeur de FFR (pour fractional flow reserve en anglais ou flux de réserve coronaire) associée à la sténose ainsi détectée, ainsi qu'un dispositif apte à déterminer automatiquement la présence d'une sténose coronarienne hémodynamiquement significative en prédisant la valeur de FFR (fractional flow reserve / flux de réserve coronaire) associée à la sténose ainsi détectée et un support lisible par ordinateur non transitoire stockant des instructions de programme lisibles par ordinateur pour déterminer automatiquement la présence d'une sténose coronarienne hémodynamiquement significative en prédisant la valeur de FFR associée à la sténose ainsi détectée.

La maladie coronaire est la deuxième cause de mortalité dans les pays développés, après le cancer. Elle affecte notamment plus de quinze millions d'américains. Elle peut se révéler de façon brutale, c'est de loin la première cause de mort subite dans le monde. On recense environ 50 000 cas de mort subite par an en France, la plupart par infarctus du myocarde. Elle peut atteindre des sujets jeunes, parfois dans la trentaine. Son incidence augmente avec le vieillissement de la population et le développement de maladies chroniques comme le diabète ou l'hypertension artérielle.

Les maladies cardiovasculaires (MCV) regroupent un certain nombre de troubles affectant le coeur et les vaisseaux sanguins comme :

- l'hypertension artérielle (élévation de la tension);
- les cardiopathies coronariennes (crise cardiaque ou infarctus);
- les maladies cérébrovasculaires (accident vasculaire cérébral);
- les artériopathies périphériques;
- l'insuffisance cardiaque;
- les cardiopathies rhumatismales;
- les cardiopathies congénitales;
- les cardiomyopathies.

Les cardiopathies coronariennes appelées également maladies coronariennes, coronaropathies, ou insuffisances coronariennes, sont des maladies obstructives des artères coronaires, qui vascularisent le coeur.

Lorsqu'elle évolue vers une sténose (rétrécissement jusqu'à l'occlusion), une lésion coronarienne entraîne une maladie coronarienne, ou coronaropathie, ou insuffisance coronarienne.

Les insuffisances coronariennes ont généralement pour conséquence une ischémie myocardique, c'est-à-dire un apport en sang insuffisant (ischémie) au muscle cardiaque (myocarde), en raison notamment d'une obstruction vasculaire.

De nombreux examens complémentaires permettent d'explorer les ischémies myocardiques, les principaux étant l'électrocardiogramme, l'épreuve d'effort, l'IRM, la scintigraphie myocardique, et la coronarographie, et plus récemment l'angioscanner coronaire.

En pratique, la classification des sténoses coronaires selon leur sévérité est réalisée le plus souvent de façon visuelle en angioscanner coronaire : elle dépend d'une segmentation qui repose sur l'extraction de la ligne centrale du vaisseau. Elle est dépendante de l'expérience du lecteur. Une sténose est considérée habituellement significative pour une réduction de diamètre d'au moins 50%, par estimation visuelle. Cette évaluation visuelle reste imprécise avec une variabilité inter-observateur substantielle.

La précision dans la classification du degré de sévérité est très liée à la qualité d'image. La qualité d'image dépend de la fréquence cardiaque, des artefacts en marche d'escalier éventuels entre deux battements, de la qualité de l'injection du produit de contraste, du niveau de bruit dans l'image et de la présence éventuelle de calcifications sévères. La dernière technologie de scanner cardiaque permet d'obtenir la meilleure qualité d'image en réduisant la plupart des artefacts cités.

Une longue expérience de lecture est nécessaire pour l'analyse d'un angio-scanner coronaire (Kerl et al., "64-Slice Multidetector-row Computed Tomography in the Diagnosis of Coronary Artery Disease: Interobserver Agreement Among Radiologists With Varied Levels of Experience on a Per-patient and Per-segment Basis." J Thorac Imaging. janv 2012;27(1):29-35.

Ces situations peuvent conduire à conclure, à tort, à une sténose coronarienne, entraînant parfois à réaliser inutilement une angiographie conventionnelle, invasive et coûteuse.

L'angioscanner coronaire (ou CCTA pour Coronary CT angiography) est une méthode plus récente, très sensible pour la détection non invasive de patients chez qui on soupçonne une coronaropathie, avec une valeur prédictive négative très élevée (généralement supérieure à 95%). Étant la méthode la plus sensible, elle tend à être utilisée dans le dépistage des maladies coronaires comme examen de première intention.

En revanche, l'angioscanner coronaire a une moins bonne spécificité (d'environ 50-70%) en raison de cas de faux-positifs fréquents. Sa valeur prédictive positive du scanner est donc plus faible. On observe les cas de faux-positifs notamment en cas de calcifications coronaires et/ou en cas d'artefacts de mouvement lors de l'acquisition des images. Ces derniers, peuvent majorer ou même créer des sténoses (inexistantes) sur l'image. Ainsi, une expertise de lecture est nécessaire pour minimiser le nombre de faux positifs. Une bonne expertise s'acquière en plusieurs années (5 ans au minimum) pour des radiologues ou cardiologues travaillant dans un centre spécialisé en imagerie cardiaque.

Le document The SCOT-HEART Investigators, "Coronary CT Angiography and 5-Year Risk of Myocardial Infarction". N Engl J Med. 6 sept 2018, décrit notamment que l'usage de l'angioscanner coronaire peut réduire le taux d'infarctus et de mortalité comparé à une évaluation standard par un test d'effort classique.

Cependant l'utilisation grandissante de l'angioscanner coronaire est en pratique freinée par le niveau d'expertise nécessaire pour une interprétation fiable dans la pratique courante.

L'arrivée de l'Intelligence Artificielle (IA) permet d'envisager de transférer certains éléments de l'expertise médicale sous forme algorithmique. Les outils d'apprentissage machine ou Machine Learning en anglais, et notamment les réseaux de neurones (RN) permettent de reproduire une expertise, ce qui est très utilisé dans le domaine de la reconnaissance d'images. C'est pourquoi de multiples projets se développent dans le domaine de l'imagerie médicale.

Ainsi, la présente invention consiste notamment à adapter une expertise dans la lecture d'angioscanner coronaire en utilisant les techniques d'IA.

La publication Zreik M et al., "A Recurrent CNN for Automatic Detection and Classification of Coronary Artery Plaque and Stenosis in Coronary CT Angiography." IEEE Trans Med Imaging. 2018 divulgue une première méthode pour la détection automatique des sténoses par apprentissage machine. Toutefois, cette méthode n'utilise que des images RMP (pour reconstructions multiplanaires) étirées, et analyse les artères par fragments de volumes, avec une classification en trois grades (normal, inférieur à 50% et supérieur à 50%) sans évaluation automatisée de la qualité d'image, et n'y associe pas d'évaluation fonctionnelle.

La demande US20150112182 divulgue la prédiction de la FFR par l'utilisation de l'Intelligence Artificielle (par apprentissage machine) à partir de n'importe quel type image médicale, d'une façon générale. Dans ce document, l'application de l'imagerie scanner est mentionné à titre d'exemple tout comme l'échographie ou l'IRM. En revanche, le présent document ne mentionne pas le type d'image scanner choisi pour l'apprentissage, l'analyse regroupée d'images multiples d'une même lésion sous plusieurs incidences, le nombre d'images pour l'analyse, et/ou l'influence de la qualité d'image.

Selon le document Tonino et al., "Fractional Flow Reserve versus Angiography for Guiding Percutaneous Coronary Intervention." N Engl J Med. 15 janv 2009; la mesure de la FFR lors d'une angiographie coronaire est un examen de référence pour déterminer si une sténose coronarienne est fonctionnellement significative, avec un seuil à 0,8. En effet, il a été montré chez des patients présentant un angor stable et qui avaient une FFR inférieure ou égale à 0,8, que l'angioplastie coronaire réduisait significativement le besoin ultérieur de revascularisation urgente. Dans le cas contraire, l'angioplastie n'apportait pas de bénéfice en terme de pronostic. Dans une autre étude, la fréquence d'évènements cardiaques majeurs après angioplastie était réduite de 28% si l'intervention était décidée selon la valeur de la FFR (inférieure ou égale à 0,8) par rapport à la seule interprétation visuelle des images d'angiographie coronaire.

L'angioscanner coronaire (CCTA) est une méthode sensible pour la détection des sténoses coronaires, permettant en pratique d'écarter une sténose coronarienne quand l'examen est normal. En revanche, l'angioscanner coronaire tend à surestimer le degré de sténose coronarienne, responsable d'une spécificité moins bonne de cet outil. La différence entre le degré visuel d'une sténose et son effet hémodynamique (donc son caractère fonctionnel) est connu et confirmé dans des études récentes comparant différentes estimations de sténoses à leur valeur FFR : dans l'essai FAME, la FFR était supérieure à 0,8 pour 63% des sténoses estimées intermédiaires (50-70%), et pour 20% des cas pour les sténoses estimées sévères visuellement (entre 70 et 99%) (Tonino et al., "Angiographic Versus Functional Severity of Coronary Artery Stenoses in the FAME Study." J Am Coll Cardiol. juin 2010.

Ainsi, un procédé non-invasif qui fournirait des informations à la fois anatomiques et fonctionnelles fiables réduirait le besoin d'une angiographie conventionnelle invasive avec éventuelle mesure invasive de FFR, serait souhaitable.

La présente invention concerne une prédiction de FFR sur analyse d'Intelligence Artificielle (IA) appliquée à des images de sténose coronaire observée en scanner sous des incidences multiples, associée ou non à une combinaison d'au moins deux critères anatomiques.

D'autres approches utilisent les techniques d'apprentissage machine pour la prédiction de la FFR en utilisant une large base de données d'anatomies coronaires générées à partir d'un modèle. Ce genre d'approche est notamment divulguée dans le document Tesche et al., « Coronary CT angiography derived morphological and functional quantitative plaque markers correlated with invasive fractional flow reserve for detecting hemodynamically significant stenosis ». J Cardiovasc Comput Tomogr. mai 2016. Toutefois, dans ce document les images de coronaires utilisées ne proviennent pas de vrais patients. Leur prédiction repose sur le principe d'hémodynamique de fluides.

Par ailleurs, le document Nakanishi et al., "Automated estimation of image quality for coronary computed tomographic angiography using machine learning." Eur Radiol. sept 2018,. décrit l'utilisation de l'apprentissage profond (deep learning) pour l'évaluation automatique de la qualité d'image en CCTA (angioscanner coronaire ou Coronary CT angiography). Toutefois, dans ce document, les examens de mauvaise qualité étaient peu nombreux en raison d'une présélection artificielle qui ne correspond pas à la pratique quotidienne. Par ailleurs, dans ce document, seules des images axiales, coronales et sagittales ont été analysées, mais pas d'images RMP. Or ce sont les images RMP qui sont la base de l'interprétation radiologique en pratique courante.

Le document Lossau et al., "Motion artifact recognition and quantification in coronary CT angiography using convolutional neural networks." Med Image Anal. févr 2019, décrit également l'utilisation de l'apprentissage profond (deep learning) pour l'évaluation automatique de la qualité d'image en CCTA (angioscanner coronaire ou Coronary CT angiography). Ce document divulgue la faisabilité de l'apprentissage profond pour quantifier les artefacts de mouvement cardiaque. En revanche, dans ce document, la qualité globale de l'image n'est pas analysée (comprenant le bruit, le contraste faible ou les calcifications importantes). C'est la qualité globale de l'image qui permet de fournir un index de confiance diagnostic tenant compte de tous les paramètres interférant sur l'image.

La présente invention concerne un procédé implémenté par ordinateur pour déterminer automatiquement la présence d'une sténose coronarienne hémodynamiquement significative en prédisant un intervalle de la valeur de FFR associée à la sténose ainsi détectée, ainsi qu'un dispositif apte à déterminer automatiquement la présence d'une sténose coronarienne hémodynamiquement significative en prédisant un intervalle de la valeur de FFR associée à la sténose ainsi détectée, et un support lisible par ordinateur non transitoire stockant des instructions de programme lisibles par ordinateur pour déterminer automatiquement la présence d'une sténose coronarienne hémodynamiquement significative en prédisant un intervalle de la valeur de FFR associée à la sténose ainsi détectée.

Les études publiées en CCTA (angioscanner coronaire ou Coronary CT angiography) reposent sur des estimations visuelles des sténoses, au seuil de 50% en diamètre. La pertinence de cette détection est très dépendante de l'observateur et de son niveau d'expérience de lecture.

A ce jour, il n'existe pas d'outil fiable de détection automatique des lésions coronaires au seuil de 50% disponible en pratique courante, en raison des multiples facteurs interférant sur l'interprétation. Ces multiples facteurs sont notamment les suivants : contraste, bruit, mouvement cardiaque ou respiratoire, variation anatomique, calcifications, qui rendent l'interprétation difficile.

Les techniques d'Intelligence Artificielle utilisent des modèles statistiques capable de reproduire une expertise, longue à acquérir. En entraînant un réseau de neurones sur des milliers d'images labellisées par un expert, il est possible d'approcher le niveau de cet expert sans modélisation *a priori.* Il est donc possible ainsi de proposer une détection automatique de sténose coronarienne avec une performance proche de celle d'un expert.

C'est ce que le Demandeur a pu valider. La capacité de détecter des sténoses dépasse ainsi 90% dans les données de validation.

C'est à partir de ce seuil d'environ 50% en diamètre qu'une lésion risque de limiter le flux coronaire. La sténose est dite dans ce cas significative. On parle alors de sténose coronarienne hémodynamiquement significative.

Au-delà de la détection de la sténose au seuil, il est très important de savoir si la sténose est hémodynamique ou non. En effet, seules les sténoses qui entraînent une chute de pression en aval de la sténose doivent être traitées mécaniquement par stent ou éventuellement par pontage coronarien.

On peut mesurer cet effet hémodynamique par mesure de la chute de pression en hyperhémie maximale. C'est la base du calcul de la FFR.

Des études ont montré qu'il y a un bénéfice à traiter mécaniquement les patients présentant une sténose coronarienne seulement si la FFR est inférieure ou égale à 0,8 traduisant une chute de pression en aval de la sténose.

C'est pourquoi il est important de pouvoir prédire un niveau de FFR inférieur ou égal à 0,8 devant une image de sténose coronarienne. Une telle prédiction permet d'éviter ainsi d'autres examens plus ou moins invasifs, coûteux et dont les performances ne sont pas toujours corrélées avec les résultats de la FFR invasive.

Si la FFR commence à baisser pour des sténoses à partir de 40-50 %, il est reconnu que le degré de sténose sur l'image ne permet pas de prédire correctement et de manière fiable la valeur de la FFR.

Entre 50 et 70 % de sténoses, deux tiers des patients présentent des FFR supérieures à 0,8, donc leurs lésions ne sont pas hémodynamiquement significatives. Entre 70 et 99 % de sténoses, 80% des patients présentent en revanche une FFR inférieure ou égale à 0,8, la sténose est donc hémodynamiquement significative.

Ainsi, en cas de sténose intermédiaire, c'est-à-dire comprise entre 50 et 80%, il est difficile de savoir s'il faut traiter ou pas le patient par un système mécanique (stent), ou par pontage. En effet, à ce jour, on ne peut déterminer le caractère hémodynamique ou non d'une sténose sur la seule imagerie anatomique.

D'autres critères anatomiques ont été proposés dans la littérature mais ils ne semblent pas être suffisamment fiables pour prédire ou non un effet hémodynamique d'une sténose.

Ces critères anatomiques comprennent notamment le degré de sténose en surface ou en diamètre, le diamètre minimum, la surface luminale minimum ou la longueur de la sténose. Cependant ces critères anatomiques n'apparaissent pas avoir été étudiés en association mais seulement de façon isolée.

Le Demandeur a pu mettre en évidence que certaines associations de critères anatomiques étaient très pertinentes pour déterminer la valeur de la FFR au-dessus ou en dessous du seuil de 0,8. Cette détermination permet notamment d'opter ou non pour un traitement par stent ou pontage.

De plus, l'apprentissage direct d'un réseau de neurones des images associées à des valeurs FFR permet aussi une telle prédiction sur des images nouvelles.

Les prédictions anatomiques et par Intelligence Artificielle apparaissent complémentaires, et leur association semble très performante.

L'imagerie de stress (échographique, par IRM ou par scanner) est utilisée pour mettre en évidence le caractère hémodynamique d'une sténose D'autres techniques basées sur l'angioscanner coronaire seul, sans utiliser l'imagerie de stress, ont également été proposées :

- utilisation des caractéristiques anatomiques de la sténose, sans obtenir de résultats probants, car les critères ont été étudiés séparément et non en association comme dans la présente invention ;
- le gradient d'atténuation au-delà de la sténose (TAG pour transluminal attenuation gradient), qui n'est pas utilisé en pratique ;
- une estimation de la FFR à partir d'un modèle de dynamique de fluides (Computational Fluid Dynamics ou CFD). Ce dernier modèle est connu sous le nom de FFR-CT (pour Fractional flow reserve-computed tomography). Les techniques de simulation requièrent des ressources informatiques importantes, les résultats de FFR-CT demandent 24h de traitement. De plus, les résultats dépendent de la qualité d'image, jusqu'à 20% des cas ont été exclus des études sur la FFR-CT en raison d'une qualité d'image insuffisante.

D'autres méthodes existent mais présentent également des inconvénients. Il s'agit par exemple des méthodes utilisant un stress pharmacologique (Adénosine, Dypiridamole, ou Dobutamine), comme l'IRM de stress, qui est coûteuse, consommatrice de temps et peu disponible. Par ailleurs, l'injection de Gadolinium pourrait exposer à des risques à long terme selon des publications récentes.

Le scanner de stress est l'application la plus récente, sans évaluation à grande échelle à l'heure actuelle. Elle induit un coût supplémentaire, une injection d'iode et une irradiation qui peut être importante en raison d'acquisitions répétitives nécessaires à l'obtention d'une courbe de perfusion.

L'imagerie de médecine nucléaire implique également une dose d'irradiation au patient liée au radiotraceur injecté.

L'échographie de stress exige un opérateur entraîné, et sa sensibilité apparaît faible.

Considérant ce qui précède, un problème que se propose de résoudre la présente invention consiste notamment à détecter automatiquement une sténose potentiellement significative avec une bonne fiabilité puis de prédire la FFR directement sur des images anatomiques, lorsque la sténose est visuellement potentiellement significative, sans avoir besoin de modélisation complexe ni d'ajout d'autre examen d'imagerie. Cela présente notamment l'avantage de pouvoir réaliser une économie considérable au niveau des systèmes de santé partout dans le monde en simplifiant grandement le parcours de soin du patient.

La solution à ce problème posé a pour premier objet un procédé implémenté par ordinateur pour déterminer la présence d'une sténose coronarienne pour un patient, comprenant :

- une étape de réception d'au moins une image médicale multiplanaire curviligne ou étirée de tomodensitométrie (scanner X) dudit patient incluant la sténose coronarienne ;
- une étape de détection de ladite sténose coronarienne sur ladite image ou sur une partie de ladite image par l'utilisation d'un premier réseau de neurones profond entrainé ;

caractérisé en ce qu'il comprend en outre :

- une étape de prédiction d'un intervalle de valeur de flux de réserve coronaire (FFR pour fractional flow reserve) par mesure manuelle, semi-automatisée et/ou automatisée d'au moins deux critères morphologiques choisis parmi :

  - le diamètre minimum de la sténose en mm ;
  - la surface minimum de la sténose en mm$^2$ ;
  - le degré de sténose coronarienne maximum exprimé en pourcentage (%) de diamètre ;
  - le degré de sténose coronarienne maximum exprimé en pourcentage (%) de surface ;
  - la longueur de la sténose en mm ; et/ou
  - la masse myocardique ou le pourcentage (%) de masse myocardique en aval de la sténose coronarienne

  et/ou
- une étape de prédiction d'un intervalle de valeur de flux de réserve coronaire (FFR pour fractional flow reserve) par utilisation d'un deuxième réseau de neurones profond entraîné, appliqué directement aux images ou parties d'images détectées.

Elle a pour deuxième objet un dispositif apte à déterminer la présence d'une sténose coronarienne pour un patient, comprenant :

- des moyens de réception d'au moins une image médicale multiplanaire curviligne de tomodensitométrie (scanner X), dudit patient incluant la sténose coronarienne ;
- des moyens de détection de ladite sténose coronarienne sur ladite image ou sur une partie de ladite image, par l'utilisation d'un premier réseau de neurones profond;

caractérisé en ce qu'il comprend en outre des moyens de prédiction d'un intervalle de valeur de flux de réserve coronaire par utilisation d'un deuxième réseau de neurones profond entraîné, appliqué directement aux images ou parties d'images détectées.

Enfin, l'invention a pour dernier objet un support lisible par ordinateur non transitoire stockant des instructions de programme lisibles par ordinateur pour déterminer la présence d'une sténose coronarienne pour un patient, comprenant l'exécution par un processeur d'instructions de programmes lisibles par ordinateur ayant pour effet de réaliser les opérations suivantes :

- la réception d'au moins une image médicale multiplanaire curviligne de tomodensitométrie (scanner X) dudit patient incluant la sténose coronarienne ;
- la détection de ladite sténose coronarienne sur ladite image ou sur une partie de ladite image par l'utilisation d'un premier réseau de neurones profond entrainé ;

caractérisé en ce qu'il engendre en outre la réalisation par ledit processeur d'une opération de prédiction d'un intervalle de valeur de flux de réserve coronaire par utilisation d'un deuxième réseau de neurones profond entraîné, appliqué directement aux images ou parties d'images détectées.

Le Demandeur a notamment pu développer un procédé qui présente des qualités permettant de détecter automatiquement une sténose coronarienne potentiellement significative d'un point de vue hémodynamique, sur la base d'une expertise de haut niveau. De plus, le procédé présente l'avantage de pouvoir prédire le caractère hémodynamique de la sténose en prédisant un seuil de FFR avec une haute probabilité. Ce système permet donc, en un seul examen d'angioscanner coronaire, de fournir des résultats fiables permettant avantageusement par la suite d'adapter une conduite thérapeutique.

Le bénéfice pour le patient par rapport aux technologies existantes est donc très important, puisqu'il apporte une confirmation diagnostique mais surtout une valeur pronostique ainsi qu'une conduite thérapeutique appropriée, sans recourir à d'autres examens. Une telle richesse d'informations permet notamment, dans un second temps, de simplifier le parcours diagnostic cardiologique de façon évidente. Par ailleurs, en réduisant le recours aux autres examens, l'économie potentielle en termes de dépenses de santé est considérable.

Dans cette description, à moins qu'il ne soit spécifié autrement, il est entendu que, lorsqu'un intervalle est donné, il inclut les bornes supérieure et inférieure dudit intervalle.

L'invention et les avantages qui en découlent seront mieux compris à la lecture de la description et des modes de réalisation non limitatifs qui suivent, illustrés au regard des dessins annexés dans lesquels :

La figure 1 représente un cliché RMP curviligne de coronaire présentant une sténose.
La figure 2 représente un cliché RMP étiré de coronaire présentant une sténose.

La figure 3 est un schéma fonctionnel illustrant les différentes étapes possibles d'un procédé selon l'invention.

La figure 4 représente certains des critères anatomiques permettant de prédire si une sténose est hémodynamique ou non.

La figure 5 illustre la détection automatique d'une sténose avec une FFR inférieure ou égale à 0,8 (FFR+) sur une image RMP curvligne par un deuxième réseau de neurones.

La figure 6 illustre la performance de la détection des lésions avec FFR inférieure ou égale à 0,8 (FFR+) par le deuxième réseau de neurones, pour 1 image donnée.

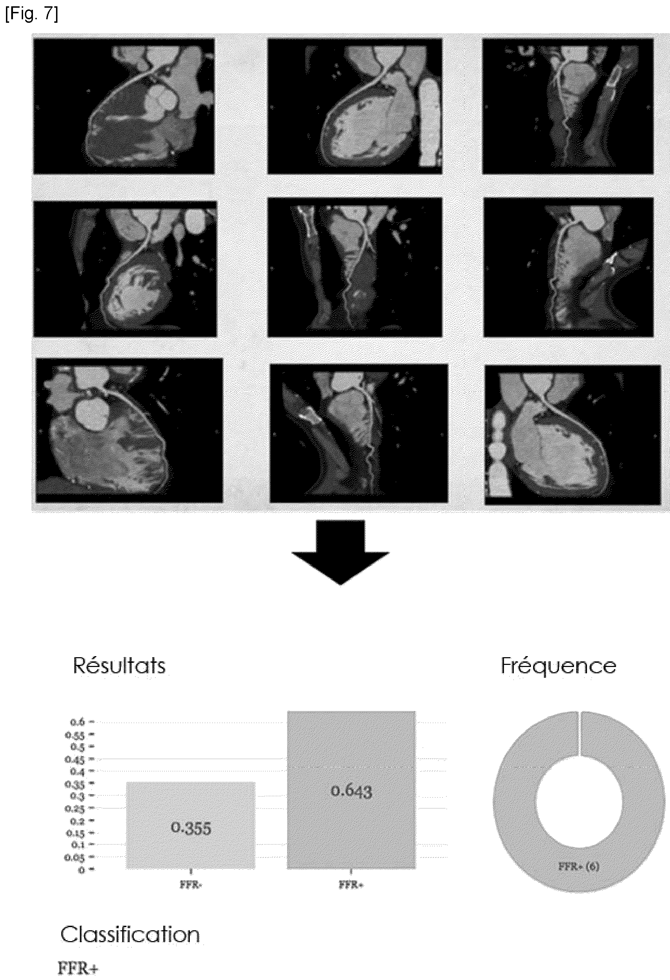

La figure 7 illustre l'analyse d'images multiples d'une même lésion observée sous des incidences différentes, permettant une classification plus robuste et plus performante de la FFR au seuil de 0,8, comparée à l'analyse d'une image isolée. Les techniques du vote majoritaire ou de la moyenne la plus élevée des classifications sont performantes. 9 images de la même artère, vue sous des incidences différentes (décalées d'au moins 20°), sont analysées successivement. Les 9 images sont classées par le réseau de neurones comme « FFR+ ». Le classement final global est donc « FFR+ » en raison du classement majoritaire (9/9) et de la moyenne la plus élevée (0,643) du classement « FFR+ ».

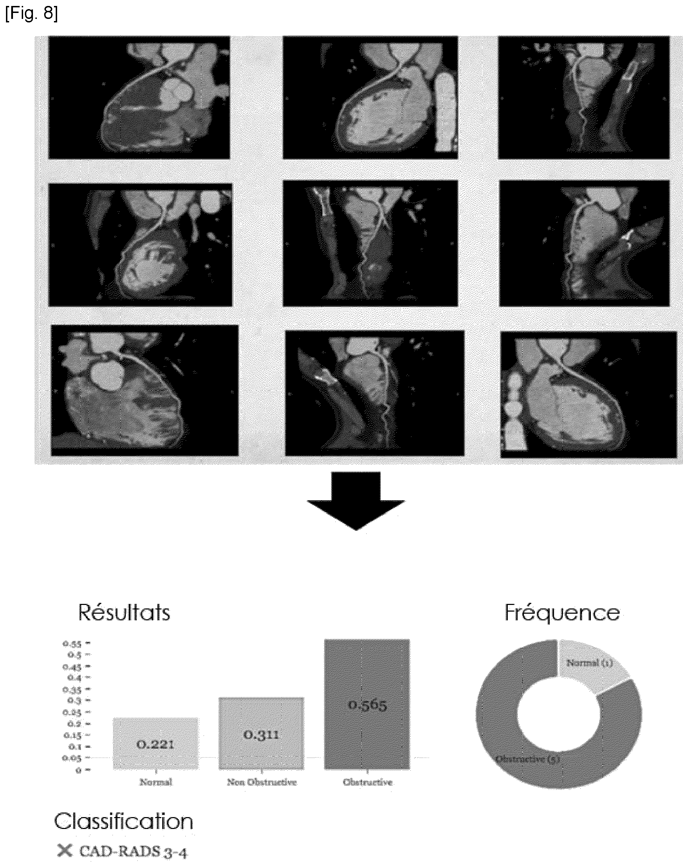

La figure 8 illustre l'analyse d'images multiples d'une même lésion observée sous des incidences différentes, permettant une classification plus robuste et plus performante selon la classification CAD-RADS, comparée à l'analyse d'une image isolée. Les techniques du vote majoritaire ou de la moyenne la plus élevée des classifications sont performantes. La figure 8 montre 9 images de la même artère, vue sous des incidences différentes (décalées d'au moins 20°), qui sont analysées successivement. 7 images sont classées par le réseau de neurones comme « normal » (CAD-RADS 0) 2 comme « obstructive » (CAD-RADS 3 ou 4). Le classement final global est donc « normal » (CAD-RADS 0) en raison du classement majoritaire (7/9) et de la moyenne la plus élevée (0,605) sur les 9 images.

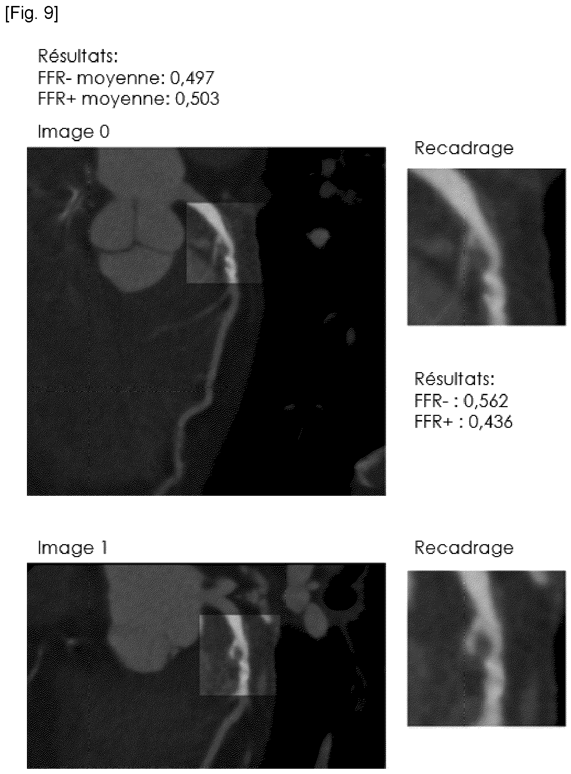

La figure 9 illustre l'analyse de fragments d'images multiples d'une même lésion observée sous des incidences différentes, permettant une classification robuste et performante de la FFR à une valeur seuil de 0,8. La zone en clair correspond au fragment d'image analysé, limitée à la lésion coronaire, permettant avantageusement une performance plus élevée d'analyse par le réseau de neurones. Ici la moyenne la plus élevée est 0,503 pour FFR +. Le classement final de la lesion est donc « FFR+ ».

L'invention a pour premier objet un procédé implémenté par ordinateur pour déterminer la présence d'une sténose coronarienne pour un patient.

La première étape dudit procédé est une étape de réception d'au moins une image médicale multiplanaire curviligne ou étirée de tomodensitométrie (scanner X) dudit patient incluant la sténose coronarienne. Une image multiplanaire est une image reconstruite à partir de la ligne centrale d'une structure anatomique tubulaire comme un vaisseau, par exemple une artère coronaire. Le grand axe du plan de l'image est alors aligné à une structure anatomique en suivant cette ligne centrale. Cela permet d'inclure la structure anatomique entière (ici une artère coronaire) dans une seule image. Une image RMP peut suivre le trajet curviligne du vaisseau, les structures adjacentes sont alors distordues. L'axe du vaisseau peut être étiré par projection selon une direction fixe. La visualisation peut se faire sur un axe de rotation de 360° dans les deux modes, curviligne ou étiré.

La deuxième étape du procédé est une étape de détection de ladite sténose coronarienne sur ladite image ou sur une partie de ladite image par l'utilisation d'un premier réseau de neurones profond.

De préférence, les images ou parties d'images sont issues d'un angioscanner coronaire (ou CCTA pour Coronary Computed Tomography Angiography).

Le premier réseau de neurones est entraîné à la lecture d'images RMP (reconstructions multiplanaires) curvilignes, images seules ou pour plus de précision des images RMP multiples de la même sténose selon plusieurs incidences, idéalement neuf incidences de 20° ou plus d'écart, afin de permettre des vues couvrant un champ minimum de 180°. Une base d'au moins 5000, préférentiellement 10000 images d'artères coronaires a été utilisée, avec des sténoses classées comme potentiellement hémodynamiquement significatives sur la base d'informations données par un expert avec plus de 20 ans d'expérience.

Les images multiplanaires curvilignes ou étirées (MPR ou reconstructions multiplanaires ou RMP) sont généralement utilisées pour l'analyse des artères coronaires. Elles sont obtenues à partir de la ligne centrale d'une artère coronaire. Cette ligne centrale est extraite par les logiciels courants sur les stations de travail radiologiques, mais il est parfois nécessaire de corriger la ligne centrale manuellement pour que cette ligne reste toujours au centre de la lumière circulante. On analyse généralement chaque artère coronaire avec des RMP multiples en multipliant les incidences sur 180 ou 360°. La méthode a été développée et validée avec neuf images avec 20° minimum d'écart, couvrant ainsi 180° au minimum. Cela permet notamment de détecter les plaques coronaires avec plus de sensibilité et de quantifier les sténoses coronaires avec plus de précision.

Une méthode connue pour la détection automatique n'utilise que les images RMP étirées, analyse les artères par fragments de volumes, avec une classification en 3 grades (normal, inférieur à 50% et supérieur à 50%), sans évaluation automatisée de la qualité d'image.

Pour les reconstructions multiplanaires (RMP), les données brutes sont tout d'abord reconstruites selon un plan perpendiculaire à l'axe z, lui-même parallèle au grand axe du patient. L'image obtenue est donc une section transversale ou axiale d'un organe tridimensionnel. À partir d'elle, il est possible de sélectionner un ou plusieurs plans de reconstruction. Les plans obtenus peuvent être arbitraires, par exemple, frontaux, coronaux et/ou sagittaux mais ils peuvent être orientés selon des axes anatomiques ou lésionnels. Un repère curviligne épousant la ligne centrale d'un vaisseau comme une artère coronaire peut permettre d'étaler cette structure selon toute sa longueur sur une reconstruction plane.

Un algorithme spécifique permet de classifier une lésion potentiellement significative hémodynamiquement à partir d'images multiples. L'algorithme, calcule la classification la plus fréquente des images d'une même artère coronaire selon différentes incidences, ainsi que la moyenne des probabilités de chaque classification. En cas de discordance de classification, le classement final retient la lésion la plus sévère (FFR+) afin de minimiser le risque de ne pas traiter à tort un patient (risque de faux négatif).

Dans les calculs de moyenne, les scores de probabilités de chaque image inférieurs à 0,2 sont exclus car considérés comme peu discriminants par le réseau de neurones.

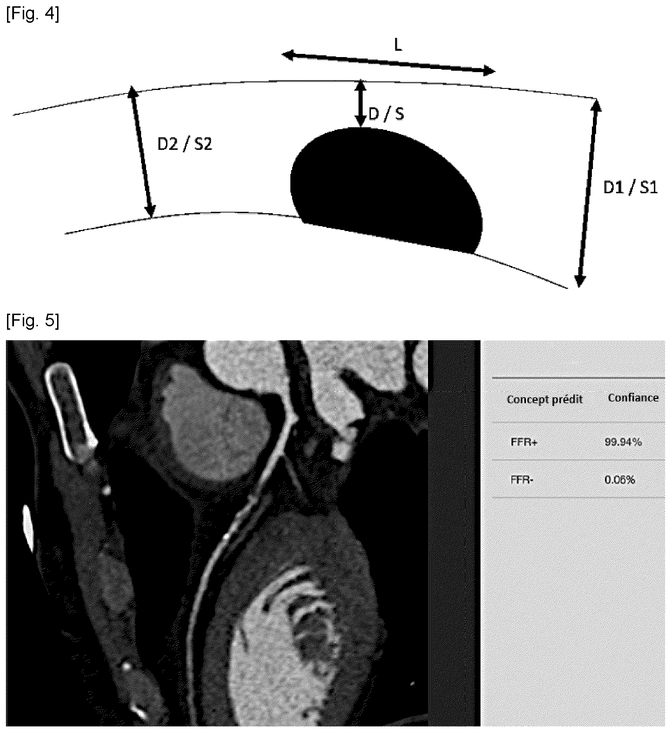

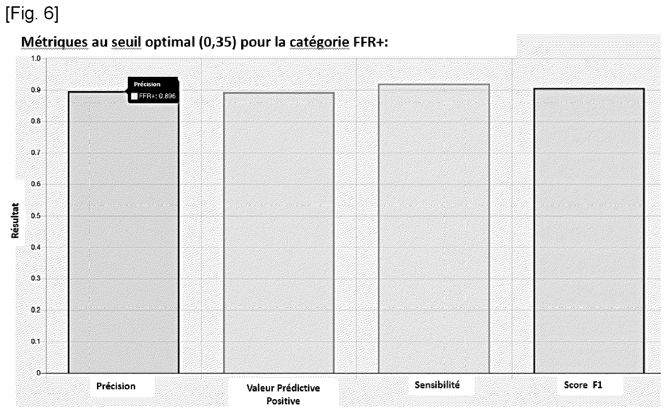

Comme il ressort notamment de la figure 5, une probabilité affichée de 0,99 par le réseau de neurones indique que la lésion est très probablement avec une FFR inférieure ou égale à 0,8, classification confirmée par les données réelles de FFR mesurée invasivement chez ce patient (FFR=0,56). Par ailleurs, comme il ressort notamment de la figure 6, au seuil de décision optimal de 0,35, la précision globale du classement est de 89,6%. Le réseau de neurones apparaît ainsi très performant pour déterminer la FFR inférieure au seuil de 0,8.

Le procédé selon l'invention comprend en outre :

- une étape de prédiction d'un intervalle de valeur de flux de réserve coronaire (FFR pour fractional flow reserve) par mesure manuelle, semi-automatisée et/ou automatisée d'au moins deux critères morphologiques choisis parmi :

  - le diamètre minimum de la sténose en mm ;
  - la surface minimum de la sténose en mm$^2$ ;
  - le degré de sténose coronarienne maximum exprimé en pourcentage (%) de diamètre ;
  - le degré de sténose coronarienne maximum exprimé en pourcentage (%) de surface ;
  - la longueur de la sténose en mm ; et/ou
  - la masse myocardique ou le pourcentage (%) de masse myocardique en aval de la sténose coronarienne ;

  et/ou

- une étape de prédiction d'un intervalle de valeur de flux de réserve coronaire par utilisation d'un deuxième réseau de neurones profond entraîné, appliqué directement aux images ou parties d'images détectées.

Selon un premier mode de réalisation de l'invention, le procédé comprend uniquement une étape additionnelle de prédiction d'un intervalle de valeur de flux de réserve coronaire par utilisation d'un deuxième réseau de neurones profond entraîné, appliqué directement aux images ou parties d'images détectées.

Selon un deuxième mode de réalisation de l'invention, le procédé comprend uniquement une étape additionnelle de prédiction d'un intervalle de valeur de flux de réserve coronaire par mesure manuelle, semi-automatisée et/ou automatisée d'au moins deux critères morphologiques choisis parmi :

- le diamètre minimum de la sténose en mm ;
- la surface minimum de la sténose en mm$^2$ ;
- le degré de sténose coronarienne maximum exprimé en pourcentage (%) de diamètre ;
- le degré de sténose coronarienne maximum exprimé en pourcentage (%) de surface ;
- la longueur de la sténose en mm ; et/ou
- la masse myocardique ou le pourcentage (%) de masse myocardique en aval de la sténose coronarienne.

Selon un troisième mode de réalisation de l'invention, le procédé comprend à la fois une étape additionnelle de prédiction d'un intervalle de valeur de flux de réserve coronaire par utilisation d'un deuxième réseau de neurones profond entraîné, appliqué directement aux images ou parties d'images détectées, et également une étape additionnelle de prédiction d'un intervalle de valeur de flux de réserve coronaire par mesure manuelle, semi-automatisée et/ou automatisée d'au moins deux critères morphologiques choisis parmi :

- le diamètre minimum de la sténose en mm ;
- la surface minimum de la sténose en mm$^2$ ;
- le degré de sténose coronarienne maximum exprimé en pourcentage (%) de diamètre ;
- le degré de sténose coronarienne maximum exprimé en pourcentage (%) de surface ;
- la longueur de la sténose en mm ; et/ou
- la masse myocardique ou le pourcentage (%) de masse myocardique en aval de la sténose coronarienne.

De préférence, lorsque la lésion est considérée potentiellement significative hémodynamiquement, alors des critères anatomiques sont extraits de l'image, de façon manuelle, semi-automatique ou automatique :

a : le diamètre minimum de la sténose en mm,
b : la surface minimum de la sténose en mm$^2$,

c : le degré de sténose coronarienne maximum exprimé en pourcentage (%) de diamètre,
d : le degré de sténose coronarienne maximum exprimé en pourcentage (%) de surface,
e : la longueur de la sténose en mm,
f : la masse myocardique ou le pourcentage (%) de masse myocardique en aval de la sténose coronarienne.

Par extraction manuelle de l'image, on entend une mesure manuelle du diamètre et de la surface minimum du vaisseau (sur une image de section d'artère) à l'endroit le plus étroit de la sténose, tracé ou contourage manuel du diamètre et de la surface (sur une image de section d'artère), au niveau d'un segment d'artère saine le plus proche de la sténose en amont et en aval de la sténose; mesure manuelle de la longueur de la sténose ; calcul de la masse myocardique en aval d'une sténose par segmentation du myocarde en aval de cette sténose ; estimation visuelle du pourcentage de myocarde en aval d'une sténose.

Par extraction semi-automatique de l'image, on entend une mesure obtenue par création préalable de lignes centrales à partir du pointage d'un vaisseau par l'utilisateur. En chaque point du vaisseau, les valeurs du diamètre minimum de la surface du vaisseau sont affichées par un algorithme sur la station de travail radiologique. Les différents paramètres d'intérêt sont lisibles au niveau de la zone d'intérêt avec possibilité de correction manuelle des lignes centrales et des contours. Des algorithmes spécifiques dédiés peuvent calculer le volume de myocarde vascularisé en aval d'une sténose (selon la console de travail radiologique dédiée).

Par extraction automatique de l'image, on entend une mesure obtenue automatiquement par création automatique des lignes et des contours du vaisseau lors du chargement des images d'un patient. Les mesures sont alors automatiquement générées par logiciel. En chaque point du vaisseau, les valeurs du diamètre minimum de la surface du vaisseau sont affichées par un algorithme sur la station de travail radiologique. Les différents paramètres d'intérêt sont lisibles au niveau de la zone d'intérêt avec possibilité de correction manuelle des lignes centrales et des contours. Des algorithmes spécifiques dédiés peuvent calculer le volume de myocarde vascularisé en aval d'une sténose (selon la console de travail radiologique dédiée).

Avantageusement, la combinaison d'au moins deux de ces critères et l'évaluation par réseau de neurones fournit une prédiction du caractère fonctionnel par la FFR au-dessus ou en dessous du seuil de 0,8.

De préférence, les critères anatomiques les plus pertinents pour prédire une valeur de FFR sont :

- la surface minimum de la sténose en mm$^2$ et
- le degré de sténose coronarienne maximum exprimé en pourcentage (%) de surface.

Plus préférentiellement encore, les critères anatomiques les plus pertinents pour prédire une valeur de FFR sont :

- la surface minimum de la sténose en mm$^2$,
- le degré de sténose coronarienne maximum exprimé en pourcentage (%) de surface, et
- la masse myocardique en aval de la sténose.

D'autres critères peuvent également être utilisés pour prédire une valeur de FFR, il s'agit des critères suivants :

- le diamètre minimum de la sténose en mm et
- le degré de sténose coronarienne maximum exprimé en pourcentage (%) de diamètre .

Comme illustré à la figure 4, les critères anatomiques permettant de qualifier une sténose sont :

- Diamètre minimum : D
- Surface minimum : S
- Degré de sténose en diamètre : D/(D1-D2/2)
- Degré de sténose en surface : S /(S1-S2/2)
- Longueur de sténose : L
- la masse myocardique ou % de masse myocardique en aval d'une sténose

Le Demandeur a pu montrer, de façon surprenante que, sur un échantillon de 120 sténoses, au moins une de ces combinaisons était notamment capable de séparer à 100% des lésions au-dessus ou en dessous du seuil de 0,83, valeur très proche du seuil cliniquement validé de 0,8.

De façon avantageuse, l'étape de prédiction d'un intervalle de valeur de flux de réserve coronaire comprend en outre l'utilisation d'un deuxième réseau de neurones profond entraîné, appliqué directement aux images ou parties d'images détectées.

Le deuxième réseau de neurones est entraîné à la lecture d'images RMP curvilignes ou étirées, images seules ou pour plus de précision des images RMP multiples de la même sténose selon plusieurs incidences. Le réseau de neurones a été entraîné avec succès sur des images de coronaires de patients pour lesquelles la valeur réelle de FFR a été mesurée de façon invasive par capteur de pression intra-coronaire.

L'analyse d'images multiples d'une sténose coronaire, vue sous des incidences différentes espacées de 20° ou plus, est avantageuse pour une meilleure classification par Intelligence Artificielle des lésions selon le seuil FFR. En effet l'aspect d'une la lésion coronaire varie selon l'incidence de visualisation : une lésion pourra être classée FFR+ selon une incidence, et FFR- selon une autre incidence. Le classement FFR+ ou FFR-d'une même image sera donc différent selon chaque incidence. Par rapport à l'analyse d'une seule image, l'analyse globale des informations FFR sur neuf incidences augmente la robustesse et la précision diagnostique (gain de précision constaté de 10% environ). Pour cela, le principe du vote majoritaire de chaque classification FFR est utilisé, et/ou la moyenne des scores de classification de chaque image (Fig. 7). Pour améliorer la robustesse, sont exclues du vote majoritaire ou de la moyenne certaines images qui n'ont pas atteint un seuil minimum de probabilité FFR pour chaque catégorie (par exemple une probabilité < à 0,2).

L'analyse par combinaison de critères anatomiques est ajoutée à l'analyse par Intelligence Artificielle. Si les deux analyses classent la lésion de la même façon, c'est ce classement qui est proposé à l'utilisateur.

En cas de discordance entre critères anatomiques et critères d'Intelligence Artificielle, c'est le classement FFR+ qui est proposé pour diminuer le risque de Faux Négatifs, car il est considéré plus grave de ne pas diagnostiquer une lésion traitable que de surévaluer une lésion (on favorise ainsi la sensibilité du diagnostic des sténoses hémodynamiques).

Ainsi, par exemple :

a/ Neuf images RMP d'une même artère avec sténose identifiée par le premier réseau de neurones sont entrées dans l'algorithme. Celui-ci trouve cinq images FFR classées « + » et quatre images FFR classées « - ». La probabilité moyenne FFR « + » est de 0,6, celle FFR « - » est de 0,5. Dans ce cas, la lésion est classée FFR « + » (hémodynamiquement significative) car le classement FFR « + » est plus fréquent et la probabilité moyenne FFR « + » est plus élevée. La lésion sera classée FFR « + » avec une probabilité de 0,6.

b/ Sept autres images RMP d'une deuxième artère avec sténose identifiée par le premier réseau de neurones sont entrées dans l'algorithme. Six sont FFR « - » avec une probabilité moyenne à 0,9, la dernière est FFR « + » avec une probabilité moyenne à 0,7. Le classement est plus fréquemment FFR « - » avec la plus forte probabilité. La sténose est alors jugée comme non hémodynamiquement significative (avec une probabilité élevée : 0,9).

Dans les calculs de moyenne, les scores de probabilités inférieurs à 0,2 sont exclus car considérés comme peu discriminants par le réseau de neurones.

Le résultat de ce deuxième réseau de neurones, s'il confirme la première prédiction sur des critères anatomiques, rend la prédiction hautement probable. En cas de discordance, le résultat de la prédiction FFR+ prime afin de privilégier la sensibilité de détection en minimisant le nombre de faux négatifs. Il est en effet considéré plus grave de sous-estimer une lésion que de la surestimer.

Le procédé selon l'invention peut avantageusement également comprendre une étape de détermination d'une valeur selon la classification CAD-RADS (pour Coronary Artery Disease - Reporting and Data System value ou système de rapports et de données) d'une sténose coronarienne par utilisation d'un troisième réseau de neurones profond entraîné, appliqué directement aux images ou parties d'images détectées.

Ledit troisième réseau de neurones a été entraîné avec succès par apprentissage supervisé sur des artères dans laquelle une classification CAD-RADS a été appliquée par un expert reconnu.

Un algorithme spécifique permet de classer une artère coronaire selon CAD-RADS à partir d'images multiples. L'algorithme, prend la classification la plus fréquente des images de différentes incidences (de 0 à 5), et la compare à la moyenne des probabilités de chaque classification. Si la classification la plus fréquente est aussi celle avec la probabilité moyenne la plus élevée, celle-ci est retenue par l'algorithme. En cas de discordance, c'est le classement le plus fréquent qui est retenu. L'algorithme élimine des calculs les scores de probabilités en dessous d'un certain seuil de décision, préalablement défini afin optimiser les performances diagnostiques du réseau de neurones. L'analyse d'images multiples d'une sténose coronaire, vue sous des incidences différentes espacées de 20° ou plus, est avantageuse pour une meilleure classification des lésions selon leur classification CAD-RADS. En effet les lésions coronaires (plaques ou sténoses), ne sont souvent pas développées de façon symétrique. Par conséquent, l'aspect de la lésion varie selon l'incidence proposée : une lésion pourra paraître serrée selon une incidence, moins serrée ou même parfois inexistante selon une troisième incidence. Le classement d'une même image sera donc différent selon son incidence. Par rapport à l'analyse d'une seule image, L'analyse globale des informations CAD-RADS sur neuf incidences augmente la robustesse et la précision (gain constaté de 10% de précision). Pour cela, le principe du vote majoritaire de chaque classification CAD-RADS par image est utilisé, et/ou la moyenne des classifications de chaque image. Pour améliorer la robustesse, sont exclus du vote ou de la moyenne certaines images qui n'ont pas atteint un seuil minimum de probabilité pour chaque catégorie CAD-RADS.

Ainsi, par exemple :

a/ Neuf images RMP d'une même artère sont classées par le troisième réseau de neurones. Cinq images RMP sont classées CAD-RADS 4, deux images RMP sont classées CAD-RADS 3, deux images RMP sont classées CAD-RADS 2. La probabilité moyenne CAD-RADS 4 est de 0,8, celle CAD-RADS 3 est de 0,5, celle de CAD-RADS 2 est de 0,3. Dans ce cas, la lésion est classée CAD-RADS 4 car ce classement est plus fréquent et sa probabilité moyenne CAD-RADS 4 est plus élevée. La lésion sera classée CAD-RADS 4 avec une probabilité de 0,8 (confiance forte).

b/ Sept autres images RMP d'une même artère sont classées par le troisième réseau de neurones. Quatre images RMP sont classées CAD-RADS 2, trois images RMP sont classées CAD-RADS 3. La probabilité moyenne CAD-RADS 2 est de 0,6, celle CAD-RADS 3 est de 0,7. Dans ce cas, la lésion est classée CAD-RADS 2 car ce classement est plus fréquent. La lésion sera classée CAD-RADS 2 avec une probabilité de 0,6 (avec une faible confiance).

Dans les calculs de moyenne, les scores de probabilités inférieurs à 0,2 pour une catégorie sont exclus car considérés comme trop peu discriminants par le réseau de neurones.

La classification CAD-RADS permet une classification rationnelle et uniformisée des lésions coronaires athéromateuses. Cette classification en 6 degrés de sévérité (de 0 à 5), permet de proposer des choix thérapeutiques optimaux pour le patient à la lumière des résultats du scanner coronaire.

A ce jour, aucune classification automatique sur la base de CAD-RADS n'a été proposée. Une telle détection automatique, si elle est fiable, est notamment très utile pour faciliter le travail quotidien d'interprétation des médecins radiologues ou cardiologues.

Avantageusement, la classification CAD-RADS renforce la valeur prédite de la FFR, car les classifications CAD-RADS inférieures à 3 correspondent aux sténoses inférieures à 50%.

Par conséquent, un score CAD-RADS inférieur à 3 n'est probablement pas associée à une FFR inférieure ou égale à 0,8.

A l'inverse, une classification CAD-RADS 4 renforce fortement la probabilité d'une valeur de FFR inférieure ou égale à 0,8.

Le procédé selon l'invention comprend ainsi avantageusement une classification CAD-RADS pour chaque image RMP curviligne d'une même artère sous différents angles.

Pour un groupe d'une à neuf images, le score moyen de probabilité de chaque catégorie CAD-RADS est calculé ainsi que la catégorie CAD-RADS la plus fréquente. Si la catégorie qui obtient le meilleur score est aussi la catégorie la plus fréquente, elle est retenue comme la classification la plus probable (Fig. 8). Sinon le résultat le plus sévère des deux classements est retenu, afin de minimiser le risque de sous-estimer une lésion. La surestimation est considérée comme plus dangereuse que la surestimation pour la détection des lésions coronariennes.

- CAD-RADS 0: normal
- CAD-RADS 1: plaque < 25%
- CAD-RADS 2: plaque entre 25 et 49%
- CAD-RADS 3: 50-69% (sténose)
- CAD-RADS 4: 70-99% (sténose)
- CAD-RADS 5: occlusion

Préférentiellement, pour une classification plus robuste les catégories 1 et 2 et les catégories 3 et 4 peuvent être regroupées de la manière suivante, selon le souhait de l'utilisateur final.

- CAD-RADS 0: normal
- CAD-RADS 1 or 2: coronaropathie non obstructive
- CAD-RADS 3 or 4: coronaropathie obstructive
- CAD-RADS 5: occlusion

Le procédé selon l'invention comprend avantageusement en outre au moins une des étapes suivantes, qui peuvent être accomplies dans n'importe quel ordre :

- une étape de détermination automatisée de la qualité d'image fournissant un index de confiance diagnostic par utilisation d'un quatrième réseau de neurones entraîné, appliqué directement aux images ou parties d'images détectées ;
- une étape de détermination d'un score de calcification global sur une échelle de 0 à 4 prédisant la catégorie du score calcique d'Agatston, par utilisation d'un cinquième réseau de neurones entraîné, appliqué directement aux images ou parties d'images détectées ; et/ou
- une étape de détermination de plaque à haut risque (PHR ou HRP pour high risk plaque) d'évènement cardiaque, par utilisation d'un sixième réseau de neurones entraîné, appliqué directement aux images ou parties d'images détectées.

Une excellente qualité d'image est avantageuse pour obtenir un diagnostic fiable, pertinent et précis en CCTA. La présence d'artefacts, liés au mouvement cardiaque, à un contraste insuffisant, ou au bruit dans l'image (le bruit est mesuré comme l'écart-type des valeurs de pixels dans une région homogène d'une image), interfèrent pour le diagnostic et la quantification d'une sténose coronarienne et rendent les décisions thérapeutiques qui s'en suivent plus difficiles. Une évaluation automatique de la qualité d'image est utile pour le contrôle de la qualité, et pour comparer les images d'un centre à l'autre. Dans la présente invention, cette évaluation automatique est avantageusement utilisée pour fournir un index de confiance diagnostique dans l'interprétation finale.

Le quatrième réseau de neurones pour l'étape de détermination automatisée de la qualité d'image a été entraîné avec succès par apprentissage supervisé sur des images RMP d'artères dont les qualités d'image ont été évaluées par un expert reconnu. Les images ont été classées selon un score de 0 à 4 selon l'échelle subjective (détaillée plus bas).

Le réseau de neurones fournit un score global de qualité d'image à partir d'une à neuf images de la même artère. La valeur moyenne des scores de qualité d'image est calculée. Cette valeur définit un index de confiance entre 0 et 4.

Un algorithme spécifique permet de classer la qualité d'image à partir d'images multiples de la même artère. L'algorithme, prend la moyenne des classifications des images d'une artère selon des différentes incidences (classées de 0 à 4).

Ainsi, par exemple :

a/ Neuf images RMP d'une même artère sont classées par le quatrième réseau de neurones. Cinq images RMP sont classée QI 4, quatre images RMP sont classées QI 3.

La qualité retenue sera (5*4+4*3)/9 =3,6. Ce chiffre est par exemple considéré comme un indicateur de confiance pour l'analyse finale.

La classification de la qualité d'image est reprise ci-dessous :

- IQ=0- Non évaluable
- IQ=1- Faible qualité d'image. Présence d'artefacts. Faible confiance diagnostique
- IQ=2. Juste. L'interprétation est possible mais le degré de confiance est peu élevé
- IQ=3. Bonne QI. Bonne confiance diagnostique
- IQ=4. Excellente QI. Haut degré de confiance

Le cinquième réseau pour l'étape de détermination d'un score de calcification global a été entraîné directement sur des images RMP d'artères pour lesquels le score d'Agatston était connu par un examen scanner préalable sans injection de produit de contraste. Après entraînement, le degré de calcification est prédit de façon semi-quantitative sur un angioscanner injecté, selon quatre catégories, pour chacune des artères extraites :

- 0 : Pas de calcification
- 1 : Calcifications modérées : score d'Agatston prédit entre 1 et 99
- 2 : Calcifications moyenne : score calcique d'Agatston prédit : entre 100 et 400
- 3 : Calcifications sévères : score calcique d'Agatston prédit : supérieur à 400

La détection du calcium coronaire est documentée dans la littérature : en particulier le Score d'Agatston est reconnu comme un marqueur de risque important et indépendant pour prédire les évènements coronaires, au même titre que des facteurs de risques connus comme le niveau élevé de cholestérol, le diabète ou l'hypertension.

Un algorithme retient le score le plus élevé sur les images d'une même artère selon des incidences multiples, puis fait la somme les scores obtenus pour chaque artère pour obtenir un score de calcification global, permettant de prédire un risque :

- Somme = 0 score calcique d'Agatston prédit : nul
- Somme = 1 score calcique d'Agatston prédit : 1-100
- Somme = 2 score calcique d'Agatston prédit : 100-200
- Somme = 3 score calcique d'Agatston prédit : 200-400
- Somme >=4 score calcique d'Agatston prédit :> 400

Dans la littérature, le score d'Agatston permet d'estimer le risque d'évènement cardio-vasculaire à 10 ans :

- 0 : risque minime,
- Inférieur à 100 : risque faible,
- 100-400 : risque intermédiaire,
- > 400 : risque élevé.

Selon le procédé de l'invention, le score calcique est calculé au préalable sur un scanner sans injection de contraste, car le contraste de haute densité interfère avec la détection du calcium. L'apprentissage machine a été utilisé pour estimer le score automatiquement sur des examens avec contraste, en ayant fourni au préalable le score obtenu sur un scanner sans contraste du même patient.

Ainsi, par exemple :
Pour un patient donné, cinq images sont analysées pour chaque artère principale (IVA pour l'artère interventriculaire antérieure, Cx pour l'artère circonflexe et CD pour la coronaire droite)

Les scores calciques sont les suivants :

- IVA : 0,0,0,0,1
- Cx : 1,1,1,2,1
- CD : 0,0,0,0,0

Ainsi, le score

$$Ca = Max(IVA)+Max(Cx)+Max(CD)$$
$$=1+2+0$$
$$=3$$

Le Score étant de 3, le score calcique prédit d'Agatston sera entre 200 et 400, correspondant à un risque intermédiaire.

Enfin le sixième réseau pour l'étape de détermination de plaque à haut risque a été entraîné avec succès par apprentissage supervisé sur des images RMP ou des images de section d'artères perpendiculaires aux images RMP (cross sectional images) dans laquelle la présence éventuelle d'une plaque vulnérable a été ou non détectée par un expert reconnu. Après entraînement, la présence d'une plaque vulnérable est affirmée selon un seuil de probabilité (entre 0 et 1) défini comme le seuil optimal pour la performance de ce réseau de neurones.

Les plaques à risques (HRP) sont caractérisées par la présence des éléments suivants : plaque de faible densité (LDP) , plaque à remodelage positif (PR) par augmentation de la paroi du vaisseau vers extérieur, présence d'une zone de densité négative au sein de la plaque (noyau lipidique).

La plaque présente le plus souvent un caractère asymétrique.

Le réseau de neurones utilise des images RMP qui peuvent être complétées éventuellement par des images de section d'artère au niveau de la plaque (telles que des CROSS SECTIONAL IMAGES), afin de conforter la précision de la détection.

Il n'existe à ce jour pas de système basé sur l'apprentissage profond (deep learning) qui détecte automatiquement les plaques à risque à partir d'images RMP.

La présence d'une plaque vulnérable est retenue si le score est supérieur au seuil ainsi défini sur au moins une des images RMP ou bien sur une image de section d'artère.

Un algorithme spécifique permet déterminer la présence d'une plaque vulnérable. En raison de son caractère asymétrique, il se peut qu'une plaque ne soit pas visible sur une ou plusieurs incidences d'images RMP en raison d'un angle de vue différent.

Ainsi, par exemple :
a/ Cinq images RMP d'une même artère sont analysées par le sixième réseau de neurones. La présence d'une plaque vulnérable est notée V, si le seuil de probabilité atteint ou dépasse 0,5, son absence est notée 0.

Le résultat 0,0,0,V,0 correspond à la présence d'une plaque vulnérable (car la présence a été détectée sur au moins une image RMP).

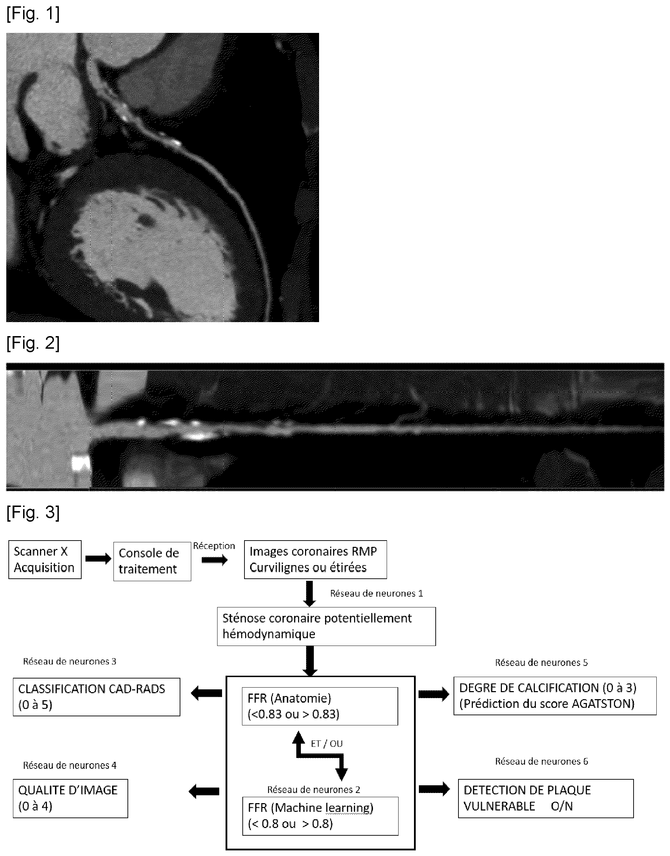

De préférence, comme cela est illustré à la figure 3, le procédé objet de l'invention comprend les cinq étapes suivantes qui peuvent être accomplies dans n'importe quel ordre :

- une étape de prédiction d'un intervalle de valeur de flux de réserve coronaire par utilisation d'un deuxième réseau de neurones profond entraîné, appliqué directement aux images ou parties d'images détectées ;
- une étape de détermination d'une valeur selon la classification CAD-RADS par utilisation d'un troisième réseau de neurones profond entraîné, appliqué directement aux images ou parties d'images détectées ;
- une étape de détermination automatisée de la qualité d'image fournissant un index de confiance diagnostic à partir d'un quatrième réseau de neurones entraîné, appliqué directement aux images ou parties d'images détectées ;
- une étape de détermination d'un score de calcification global sur une échelle de 0 à 4 prédisant le score calcique d'Agatston, à l'aide d'un cinquième réseau de neurones entraîné, appliqué directement aux images ou parties d'images détectées ; et
- une étape de détermination de plaque à haut risque d'évènement cardiaque, à l'aide d'un sixième réseau de neurones entraîné, appliqué directement aux images ou parties d'images détectées.

L'invention a également pour objet un dispositif apte à déterminer la présence d'une sténose coronarienne pour un patient, comprenant :

- des moyens de réception d'au moins une image médicale multiplanaire curviligne de tomodensitométrie (scanner X), dudit patient incluant la sténose coronarienne ;
- des moyens de détection de ladite sténose coronarienne sur ladite image ou sur une partie de ladite image, par l'utilisation d'un premier réseau de neurones profond.

Ledit dispositif comprend en outre des moyens de prédiction d'un intervalle de valeur de flux de réserve coronaire par utilisation d'un deuxième réseau de neurones profond entraîné appliqué directement aux images ou parties d'images détectées.

Les premier et deuxième réseaux de neurones sont tels que décrits ci-dessus.

De préférence, le dispositif comprend en outre des moyens de détermination d'une valeur selon la classification CAD-RADS par utilisation d'un troisième réseau de neurones profond entraîné, appliqué directement aux images ou parties d'images détectées.

Le troisième réseau de neurones est tel que décrit ci-dessus.

De façon avantageuse, le dispositif selon l'invention comprend en outre au moins un des moyens suivants :

- des moyens de détermination automatisés de la qualité d'image fournissant un index de confiance diagnostic par utilisation d'un quatrième réseau de neurones entraîné, appliqué directement aux images ou parties d'images détectées ;
- des moyens de détermination d'un score de calcification global sur une échelle de 0 à 4 prédisant le score calcique d'Agatston, par utilisation d'un cinquième réseau de neurones entraîné, appliqué directement aux images ou parties d'images détectées ; et/ou
- des moyens de détermination de plaque à haut risque d'évènement cardiaque, par utilisation d'un sixième réseau de neurones entraîné, appliqué directement aux images ou parties d'images détectées.

Les quatrième, cinquième et sixième réseaux de neurones sont tels que décrits ci-dessus.

Selon un mode préféré de réalisation de l'invention, le dispositif comprend les cinq moyens suivants :

- des moyens de prédiction d'un intervalle de valeur de flux de réserve coronaire par utilisation d'un deuxième réseau de neurones profond entraîné, appliqué directement aux images ou parties d'images détectées ;
- des moyens de détermination d'une valeur selon la classification CAD-RADS par utilisation d'un troisième réseau de neurones profond entraîné, appliqué directement aux images ou parties d'images détectées ;
- des moyens de détermination automatisés de la qualité d'image fournissant un index de confiance diagnostic par utilisation d'un quatrième réseau de neurones entraîné, appliqué directement aux images ou parties d'images détectées ;
- des moyens de détermination d'un score de calcification global sur une échelle de 0 à 4 prédisant le score calcique d'Agatston, par utilisation d'un cinquième réseau de neurones entraîné, appliqué directement aux images ou parties d'images détectées ; et
- des moyens de détermination de plaque à haut risque d'évènement cardiaque, par utilisation d'un sixième réseau de neurones entraîné, appliqué directement aux images ou parties d'images détectées.

Enfin, l'invention a pour objet un support lisible par ordinateur non transitoire stockant des instructions de programme lisibles par ordinateur pour déterminer la présence d'une sténose coronarienne pour un patient, comprenant l'exécution par un processeur d'instructions de programmes lisibles par ordinateur ayant pour effet de réaliser les opérations suivantes:

- la réception d'au moins une image médicale multiplanaire curviligne de tomodensitométrie (scanner X) dudit patient incluant la sténose coronarienne ;
- la détection de ladite sténose coronarienne potentiellement hémodynamiquement significative sur ladite image ou sur une partie de ladite image par l'utilisation d'un premier réseau de neurones profond entrainé ;

caractérisé en ce qu'il engendre en outre la réalisation par ledit processeur d'une opération de prédiction d'un intervalle de valeur de flux de réserve coronaire par utilisation d'un deuxième réseau de neurones profond entraîné, appliqué directement aux images ou parties d'images détectées.

De préférence, le support est apte à engendrer en outre la réalisation par ledit processeur d'une opération de détermination d'une valeur selon la classification CAD-RADS par utilisation d'un troisième réseau de neurones profond entraîné, appliqué directement aux images ou parties d'images détectées.

De préférence encore, le support est apte à engendrer en outre la réalisation par ledit processeur d'au moins une des opérations suivantes de :

- détermination automatisée de la qualité d'image fournissant un index de confiance diagnostic par utilisation d'un quatrième réseau de neurones entraîné, appliqué directement aux images ou parties d'images détectées ;
- détermination d'un score de calcification global sur une échelle de 0 à 4 prédisant le score calcique d'Agatston, par utilisation d'un cinquième réseau de neurones entraîné, appliqué directement aux images ou parties d'images détectées ; et/ou

- détermination de plaque à haut risque d'évènement cardiaque, par utilisation d'un sixième réseau de neurones entraîné, appliqué directement aux images ou parties d'images détectées.

Selon un mode préféré de réalisation de l'invention, le support engendre la réalisation par ledit processeur des cinq opérations suivantes :

- prédiction d'un intervalle de valeur de flux de réserve coronaire par utilisation d'un deuxième réseau de neurones profond entraîné, appliqué directement aux images ou parties d'images détectées ;
- détermination d'une valeur selon la classification CAD-RADS par utilisation d'un troisième réseau de neurones profond entraîné, appliqué directement aux images ou parties d'images détectées ;
- détermination automatisée de la qualité d'image fournissant un index de confiance diagnostic par utilisation d'un quatrième réseau de neurones entraîné, appliqué directement aux images ou parties d'images détectées ;
- détermination d'un score de calcification global sur une échelle de 0 à 4 prédisant le score calcique d'Agatston, par utilisation d'un cinquième réseau de neurones entraîné, appliqué directement aux images ou parties d'images détectées ; et
- détermination de plaque à haut risque d'évènement cardiaque, par utilisation d'un sixième réseau de neurones entraîné, appliqué directement aux images ou parties d'images détectées.

La présente invention va maintenant être illustrée au moyens des exemples suivants :

Exemple 1 :

Une large base de données de plus d'au moins 5000, de préférence plus de 10000 images RMP issues d'angioscanners coronaires a été utilisée pour un apprentissage supervisé. Toutes les images ont été classées et labellisées par un expert avec plus de 20 ans d'expérience de lecture de ces images (expérience cumulée d'environ 50.000 cas analysés). Les images de cette base ont été classées en terme de qualité d'image, de degré de calcification, et de degré de sténose en fonction de la classification CAD-RADS. La présence éventuelle de plaque à risque (plaque vulnérable) a été précisée. De plus, sur plus de 2000, préférentiellement 4000 images de patients avec sténose et valeur de FFR connue, une classification binaire au seuil FFR de 0,8 a été effectuée. Un réseau de neurones a pu être ainsi entraîné (sur 80% des images) pour prédire sur une nouvelle image (parmi les 20% des images restantes, servant de base de test) si la valeur de FFR sera supérieure ou inférieure (> ou <) à 0,8.

Différents réseaux de neurones disponibles en accès libre ont été testés : GOOGLENET™, RESNET™ et INCEPTION™ V3, VGG11™, VGG13™, VGG19™ afin d'obtenir le meilleur taux de classification. Diverses mesures ont été effectuées sur une base de données test, indépendante de la base d'apprentissage : calculs de précision du test, de sensibilité, de spécificité, de valeur prédictive positive, de score F1 (moyenne harmonique entre la sensibilité et la valeur prédictive positive), aire sous la courbe ROC (Receiving Operator Curve, avec sensibilité en abscisse et 1-spécificité en ordonnée).

Une aire sous la courbe atteignant 0,85 a été obtenue, traduisant une bonne performance prédictive globale, égale ou supérieure aux systèmes prédictifs actuels. Les scores devraient progresser avec l'augmentation progressive de la base d'entraînement.

Les images multiplanaires RMP sont fournies de base par les consoles de post-traitement radiologique, à partir des lignes centrales. Images RMP curviligne ou étirées, une à neuf images de la même artère. Les images peuvent être exportées des stations de travail sous un format d'image standard (ex: JPEG, ou PNG), et chargées secondairement sur un site Internet dédié. Elles peuvent aussi être directement chargées sur un site Internet à partir de la station de travail au format radiologique standard DICOM. Le résultat d'évaluation produit est alors retourné dans l'environnement habituel du lecteur. Les réseaux de neurones pourront être aussi directement intégrés aux consoles de post-traitement par dockerisation.

Exemple 2 : Création de réseaux de neurones, méthodes et résultats

Une large base de données d'image RMP étirées ou curviligne a été créé à partir d'examens réalisés sur différents scanners.

Les données des patients ont été anonymisées.

Pour chaque jeu de données les images RMP des trois artères principales ont été extraites, à savoir : IVA, la circonflexe gauche, et l'artère coronaire droite.

Pour chacune des trois artères, neuf images ont été sélectionnées avec des angles de vue différents (20° d'écart minimum entre 2 images), par rotation autour de la ligne centrale.

Chaque image RMP a été classifiée par un expert au regard de la qualité d'image, du degré de calcification, de la présence ou non d'une plaque vulnérable, et de son degré de sténose en utilisant la classification CAD-RADS.

Une base de données réduite de plus de 1800, préférentiellement 4500 images sur plus de 200, préférentiellement 500 patients avec une valeur de FFR connue a été aussi entraînée.

Les images ont été chargées sur une plateforme (DEEPOMATIC™ STUDIO, Paris, France et CLEVERDOC, Lille, France) permettant de classifier les images et de tester les différents réseaux de neurones.

Pour chaque réseau de neurones, 80 % des images de la base de données ont été utilisées pour l'entraînement, et 20% des images ont été utilisées pour l'évaluation. Les images d'évaluation ont été exclues du processus d'entraînement.

Pour chaque tâche, différents réseaux de neurones ont été utilisés pour l'entraînement.

Les réseaux associés aux meilleurs résultats (meilleure sensibilité et meilleur valeur prédictive positive) ont été sélectionnés.

Résultats et conclusion :

Pour la détection automatique des plaques vulnérables le score F1 moyen a atteint 60 %.

Pour le score calcique automatique le score F1 moyen atteint 75 %.

Pour la classification automatique de la qualité d'image le score F1 atteint 75 %.

Pour la classification des sténoses selon CAD-RADS le score F1 moyen atteint 87 %.

Pour la prédiction de la FFR au seuil de 0,8, le score F1 moyen atteint environ 85 % voire 87%.

Les résultats obtenus sur cette première base semblent donc égaux ou supérieurs à ceux des autres méthodes qui sont plus complexes à mettre en œuvre et plus coûteuses.

**Revendications**

1. Procédé implémenté par ordinateur pour déterminer la présence d'une sténose coronarienne pour un patient, comprenant :

    - une étape de réception d' images médicales multiplanaires curvilignes ou étirées de tomodensitométrie (scanner X) incluant la sténose coronarienne dudit patient, lesdites images incluant des représentations de la sténose coronarienne vue sous des incidences différentes ;
    - une étape de détection de ladite sténose coronarienne sur lesdites images ou sur une partie desdites images par l'utilisation d'un premier réseau de neurones profond entrainé à la lecture desdites images ;
    - une étape de prédiction d'un intervalle de valeur de flux de réserve coronaire (FFR pour fractional flow reserve) par utilisation d'un deuxième réseau de neurones profond entraîné, appliqué directement auxdites images détectées ou parties desdites images détectées.

2. Procédé selon la revendication 1, **caractérisé en ce qu'**il comprend en outre une étape de détermination d'une valeur selon la classification CAD-RADS (pour Coronary Artery Disease - Reporting and Data System value ou système de rapports et de données) d'une sténose coronarienne par utilisation d'un troisième réseau de neurones profond entraîné, appliqué directement aux images ou parties d'images détectées.

3. Procédé selon la revendication 1 ou 2, **caractérisé en ce qu'**il comprend en outre au moins une des étapes suivantes, qui peuvent être accomplies dans n'importe quel ordre :

    - une étape de détermination automatisée de la qualité d'image fournissant un index de confiance diagnostic par utilisation d'un quatrième réseau de neurones entraîné, appliqué directement aux images ou parties d'images détectées ;
    - une étape de détermination d'un score de calcification global sur une échelle de 0 à 4 prédisant la catégorie du score calcique d'Agatston, par utilisation d'un cinquième réseau de neurones entraîné, appliqué directement aux images ou parties d'images détectées ; et/ou
    - une étape de détermination de plaque à haut risque (PHR ou HRP pour high risk plaque) d'évènement cardiaque, par utilisation d'un sixième réseau de neurones entraîné, appliqué directement aux images ou parties d'images détectées.

4. Procédé selon la revendication 3, **caractérisé en ce qu'**il comprend en outre une étape de prédiction d'un intervalle de valeur de flux de réserve coronaire (FFR pour fractional flow reserve) par mesure manuelle, semi-automatisée et/ou automatisée d'au moins deux critères morphologiques choisis parmi :

    • le diamètre minimum de la sténose en mm ;
    • la surface minimum de la sténose en mm$^2$ ;
    • le degré de sténose coronarienne maximum exprimé en pourcentage (%) de diamètre ;
    • le degré de sténose coronarienne maximum exprimé en pourcentage (%) de surface ;
    • la longueur de la sténose en mm ; et/ou
    • la masse myocardique ou le pourcentage (%) de masse myocardique en aval de la sténose coronarienne.

5. Procédé selon l'une des revendications précédentes, **caractérisé en ce que** les images ou parties d'images sont issues d'un angioscanner coronaire (ou CCTA pour Coronary Computed Tomography Angiography).

6. Dispositif apte à déterminer la présence d'une sténose coronarienne pour un patient, comprenant :

    - des moyens de réception d'images médicales multiplanaires curvilignes de tomodensitométrie (scanner X) incluant la sténose coronarienne dudit patient, lesdites images incluant des représentations de la sténose coronarienne vue sous des incidences différentes ;
- des moyens de détection de ladite sténose coronarienne sur lesdites images ou sur une partie desdites images, par l'utilisation d'un premier réseau de neurones profond entrainé à la lecture desdites images ;

**caractérisé en ce qu'**il comprend en outre des moyens de prédiction d'un intervalle de valeur de flux de réserve coronaire par utilisation d'un deuxième réseau de neurones profond entraîné, appliqué directement auxdites images détectées ou parties desdites images détectées.

7. Dispositif selon la revendication 6, **caractérisé en ce qu'**il comprend en outre des moyens de détermination d'une valeur selon la classification CAD-RADS par utilisation d'un troisième réseau de neurones profond entraîné, appliqué directement aux images ou parties d'images détectées.

8. Dispositif selon l'une des revendications 6 ou 7, **caractérisé en ce qu'**il comprend en outre au moins un des moyens suivants :

   - des moyens de détermination automatisés de la qualité d'image fournissant un index de confiance diagnostic par utilisation d'un quatrième réseau de neurones entraîné, appliqué directement aux images ou parties d'images détectées ;
   - des moyens de détermination d'un score de calcification global sur une échelle de 0 à 4 prédisant le score calcique d'Agatston, par utilisation d'un cinquième réseau de neurones entraîné, appliqué directement aux images ou parties d'images détectées ; et/ou
   - des moyens de détermination de plaque à haut risque d'évènement cardiaque, par utilisation d'un sixième réseau de neurones entraîné, appliqué directement aux images ou parties d'images détectées.

9. Dispositif selon la revendication 8, **caractérisé en ce qu'**il comprend les cinq moyens suivants :

   - des moyens de prédiction d'un intervalle de valeur de flux de réserve coronaire par utilisation d'un deuxième réseau de neurones profond entraîné, appliqué directement aux images ou parties d'images détectées ;
   - des moyens de détermination d'une valeur selon la classification CAD-RADS par utilisation d'un troisième réseau de neurones profond entraîné, appliqué directement aux images ou parties d'images détectées ;
- des moyens de détermination automatisés de la qualité d'image fournissant un index de confiance diagnostic par utilisation d'un quatrième réseau de neurones entraîné, appliqué directement aux images ou parties d'images détectées ;
- des moyens de détermination d'un score de calcification global sur une échelle de 0 à 4 prédisant le score calcique d'Agatston, par utilisation d'un cinquième réseau de neurones entraîné, appliqué directement aux images ou parties d'images détectées ; et
- des moyens de détermination de plaque à haut risque d'évènement cardiaque, par utilisation d'un sixième réseau de neurones entraîné, appliqué directement aux images ou parties d'images détectées.

10. Support lisible par ordinateur non transitoire stockant des instructions de programme lisibles par ordinateur pour déterminer la présence d'une sténose coronarienne pour un patient, comprenant l'exécution par un processeur d'instructions de programmes lisibles par ordinateur ayant pour effet de réaliser les opérations suivantes :

    - la réception des images médicales multiplanaires curvilignes de tomodensitométrie (scanner X) incluant la sténose coronarienne dudit patient ; lesdites images incluant des représentations de la sténose coronarienne vue sous des incidences différentes ;
    - la détection de ladite sténose coronarienne potentiellement hémodynamiquement significative sur lesdites image ou sur une partie desdites images par l'utilisation d'un premier réseau de neurones profond entrainé à la lecture desdites images ;

    **caractérisé en ce qu'**il engendre en outre la réalisation par ledit processeur d'une opération de prédiction d'un intervalle de valeur de flux de réserve coronaire par utilisation d'un deuxième réseau de neurones profond entraîné, appliqué directement auxdites images détectées ou parties desdites images détectées.

11. Support selon la revendication 10, **caractérisé en ce qu'**il engendre en outre la réalisation par ledit processeur d'une opération de détermination d'une valeur selon la classification CAD-RADS par utilisation d'un troisième réseau de neurones profond entraîné, appliqué directement aux images ou parties d'images détectées.

12. Support selon l'une des revendications 10 ou 11, **caractérisé en ce qu'**il engendre en outre la réalisation par ledit processeur d'au moins une des opérations suivantes de :

- détermination automatisée de la qualité d'image fournissant un index de confiance diagnostic par utilisation d'un quatrième réseau de neurones entraîné, appliqué directement aux images ou parties d'images détectées ;
- détermination d'un score de calcification global sur une échelle de 0 à 4 prédisant le score calcique d'Agatston, par utilisation d'un cinquième réseau de neurones entraîné, appliqué directement aux images ou parties d'images détectées ; et/ou
- détermination de plaque à haut risque d'évènement cardiaque, par utilisation d'un sixième réseau de neurones entraîné, appliqué directement aux images ou parties d'images détectées.

13. Support selon la revendication 12, **caractérisé en ce qu'**il engendre la réalisation par ledit processeur des cinq opérations suivantes :

- prédiction d'un intervalle de valeur de flux de réserve coronaire par utilisation d'un deuxième réseau de neurones profond entraîné, appliqué directement aux images ou parties d'images détectées ;
- détermination d'une valeur selon la classification CAD-RADS par utilisation d'un troisième réseau de neurones profond entraîné, appliqué directement aux images ou parties d'images détectées ;
- détermination automatisée de la qualité d'image fournissant un index de confiance diagnostic par utilisation d'un quatrième réseau de neurones entraîné, appliqué directement aux images ou parties d'images détectées ;
- détermination d'un score de calcification global sur une échelle de 0 à 4 prédisant le score calcique d'Agatston, par utilisation d'un cinquième réseau de neurones entraîné, appliqué directement aux images ou parties d'images détectées ; et
- détermination de plaque à haut risque d'évènement cardiaque, par utilisation d'un sixième réseau de neurones entraîné, appliqué directement aux images ou parties d'images détectées.

**Patentansprüche**

1. Computerimplementiertes Verfahren zum Bestimmen des Vorhandenseins einer Koronarstenose bei einem Patienten, das Folgendes umfasst:

- einen Schritt des Empfangens von multiplanaren gekrümmten oder gestreckten medizinischen CT-Bildern (Röntgenscanner), die die Koronarstenose des Patienten einschließen, wobei die Bilder Darstellungen der Koronarstenose umfassen, die unter verschiedenen Einflüssen gesehen werden;
- einen Schritt des Detektierens der Koronarstenose auf den Bildern oder auf einem Teil der Bilder unter Verwendung eines ersten tiefen neuronalen Netzes, das auf das Lesen der Bilder trainiert ist;
- einen Schritt des Vorhersagens eines Intervalls für den Wert des Koronarreserveflusses (FFR für fraktionelle Flussreserve) unter Verwendung eines zweiten trainierten tiefen neuronalen Netzes, das direkt auf die detektierten Bilder oder Teile der detektierten Bilder angewendet wird.

2. Verfahren nach Anspruch 1, **dadurch gekennzeichnet, dass** es ferner einen Schritt des Bestimmens eines Wertes gemäß der CAD-RADS-Klassifizierung (für Wert von Coronary Artery Disease - Reporting and Data System oder Berichts- und Datensystem) einer Koronarstenose unter Verwendung eines dritten trainierten tiefen neuronalen Netzes umfasst, das direkt auf die detektierten Bilder oder Teile der detektierten Bilder angewendet wird.

3. Verfahren nach Anspruch 1 oder 2, **dadurch gekennzeichnet, dass** es ferner mindestens einen der folgenden Schritte umfasst, die in beliebiger Reihenfolge durchgeführt werden können:

- einen Schritt des automatisierten Bestimmens der Bildqualität, die einen diagnostischen Vertrauensindex bereitstellt, unter Verwendung eines vierten trainierten neuronalen Netzes, das direkt auf die detektierten Bilder oder Teile der detektierten Bilder angewendet wird;
- einen Schritt des Bestimmens eines globalen Kalzifizierungsscores auf einer Skala von 0 bis 4, der die Kategorie des Agatston-Kalzifizierungsscore vorhersagt, unter Verwendung eines fünften trainierten neuronalen Netzes, das direkt auf die detektierten Bilder oder Teile der detektierten Bilder angewendet wird; und/oder
- einen Schritt des Bestimmens von Plaque mit hohem Risiko (PHR oder HRP für High Risk Plaque) für ein kardiales Ereignis unter Verwendung eines sechsten trainierten neuronalen Netzes, das direkt auf die detektierten Bilder oder Teile der detektierten Bilder angewendet wird.

4. Verfahren nach Anspruch 3, **dadurch gekennzeichnet, dass** es ferner einen Schritt des Vorhersagens eines Intervalls für den Wert des Koronarreserveflusses (FFR für fraktionelle Flussreserve) durch manuelle, halbautomatisierte und/oder automatisierte Messung von mindestens zwei morphologischen Kriterien umfasst, die aus Folgendem ausgewählt sind:

• dem Mindestdurchmesser der Stenose in mm
• der Mindestfläche der Stenose in mm$^2$
• dem maximalen Grad der Koronarstenose, ausgedrückt in Prozent (%) des Durchmessers
• dem maximalen Grad der Koronarstenose, ausgedrückt in Prozent (%) der Fläche
• der Länge der Stenose in mm; und/oder
• der Myokardmasse oder dem Prozentsatz (%) der Myokardmasse nach der Koronarstenose.

5. Verfahren nach einem der vorhergehenden Ansprüche, **dadurch gekennzeichnet, dass** die Bilder oder Teile von Bildern aus einem Koronar-Angioscanner (oder CCTA für Coronary Computed Tomography Angiography) stammen.

6. Vorrichtung, die zum Bestimmen des Vorhandenseins einer Koronarstenose bei einem Patienten geeignet ist und Folgendes umfasst:

- Mittel zum Empfangen von multiplanaren gekrümmten medizinischen CT-Bildern (Röntgenscanner), die die Koronarstenose des Patienten einschließen, wobei die Bilder Darstellungen der Koronarstenose umfassen, die unter verschiedenen Einflüssen gesehen werden;
- Mittel zum Detektieren der Koronarstenose auf den Bildern oder auf einem Teil der Bilder unter Verwendung eines ersten tiefen neuronalen Netzes, das auf das Lesen der Bilder trainiert ist;

**dadurch gekennzeichnet, dass** es ferner Mittel zum Vorhersagen eines Intervalls für den Wert des Koronarreservefluss unter Verwendung eines zweiten trainierten tiefen neuronalen Netzes umfasst, das direkt auf die detektierten Bilder oder Teile der detektierten Bilder angewendet wird.

7. Vorrichtung nach Anspruch 6, **dadurch gekennzeichnet, dass** sie ferner Mittel zum Bestimmen eines Wertes gemäß der CAD-RADS-Klassifizierung unter Verwendung eines dritten trainierten tiefen neuronalen Netzes umfasst, das direkt auf die detektierten Bilder oder Teile der detektierten Bilder angewendet wird.

8. Vorrichtung nach einem der Ansprüche 6 oder 7, **dadurch gekennzeichnet, dass** sie ferner mindestens eines der folgenden Mittel umfasst:

- automatisierte Mittel zum Bestimmen der Bildqualität, die einen diagnostischen Vertrauensindex bereitstellt, unter Verwendung eines vierten trainierten neuronalen Netzes, das direkt auf die detektierten Bilder oder Teile der detektierten Bildern angewendet wird;
- Mittel zum Bestimmen eines globalen Kalzifizierungsscores auf einer Skala von 0 bis 4, der den Agatston-Kalzifizierungsscore vorhersagt, unter Verwendung eines fünften trainierten neuronalen Netzes, das direkt auf die detektierten Bilder oder Teile der detektierten Bilder angewendet wird; und/oder
- Mittel zum Bestimmen von Plaque mit hohem Risiko für ein kardiales Ereignis unter Verwendung eines sechsten trainierten neuronalen Netzes, das direkt auf die detektierten Bilder oder Teile der detektierten Bilder angewendet wird.

9. Vorrichtung nach Anspruch 8, **dadurch gekennzeichnet, dass** sie die folgenden fünf Mittel umfasst:

- Mittel zum Vorhersagen eines Intervalls für den Wert des Koronarreservefluss unter Verwendung eines zweiten trainierten tiefen neuronalen Netzes, das direkt auf die detektierten Bilder oder Teile der detektierten Bilder angewendet wird;
- Mittel zum Bestimmen eines Wertes gemäß der CAD-RADS-Klassifizierung unter Verwendung eines dritten trainierten tiefen neuronalen Netzes, das direkt auf die detektierten Bilder oder Teile der detektierten Bilder angewendet wird;
- automatisierte Mittel zum Bestimmen der Bildqualität, die einen diagnostischen Vertrauensindex bereitstellt, unter Verwendung eines vierten trainierten neuronalen Netzes, das direkt auf die detektierten Bilder oder Teile der detektierten Bildern angewendet wird;
- Mittel zum Bestimmen eines globalen Kalzifizierungsscores auf einer Skala von 0 bis 4, der den Agatston-Kalzifizierungsscore vorhersagt, unter Verwendung eines fünften trainierten neuronalen Netzes, das direkt auf die detektierten Bilder oder Teile der detektierten Bilder angewendet wird; und
- Mittel zum Bestimmen von Plaque mit hohem Risiko für ein kardiales Ereignis unter Verwendung eines sechsten trainierten neuronalen Netzes, das direkt auf die detektierten Bilder oder Teile der detektierten Bilder angewendet wird.

10. Nicht vorübergehendes computerlesbares Medium, das computerlesbare Programmanweisungen speichert, um das Vorhandensein einer Koronarstenose bei einem Patienten zu bestimmen, umfassend das Ausführen von computerlesbaren Programmanweisungen durch einen Prozessor, die Folgendes bewirken:

- Empfangen der multiplanaren gekrümmten medizinischen CT-Bilder (Röntgenscanner), die die Koronarstenose des Patienten einschließen; wobei die Bilder Darstellungen der Koronarstenose umfassen, die unter verschiedenen Einflüssen gesehen werden;
- Detektieren der potenziell hämodynamisch signifikanten Koronarstenose auf den Bildern oder auf einem Teil der Bilder durch die Verwendung eines ersten tiefen neuronalen Netzes, das auf das Lesen der Bilder trainiert ist;

**dadurch gekennzeichnet, dass** es ferner bewirkt, dass der Prozessor einen Vorgang des Vorhersagens eines Intervalls für den Wert des Koronarreservefluss unter Verwendung eines zweiten trainierten tiefen neuronalen Netzes durchführt, das direkt auf die detektierten Bilder oder Teile der detektierten Bilder angewendet wird.

11. Medium nach Anspruch 10, **dadurch gekennzeichnet, dass** es ferner das Durchführen eines Vorgangs durch den Prozessor zum Bestimmen eines Werts gemäß der CAD-RADS-Klassifizierung unter Verwendung eines dritten trainierten tiefen neuronalen Netzes bewirkt, das direkt auf die detektierten Bilder oder Teile der detektierten Bilder angewendet wird.

12. Medium nach einem der Ansprüche 10 oder 11, **dadurch gekennzeichnet, dass** es ferner das Durchführen mindestens eines der folgenden Vorgänge durch den Prozessor bewirkt:

- automatisiertes Bestimmen der Bildqualität, die einen diagnostischen Vertrauensindex bereitstellt, unter Verwendung eines vierten trainierten neuronalen Netzes, das direkt auf die detektierten Bilder oder Teile der detektierten Bildern angewendet wird;
- Bestimmen eines globalen Kalzifizierungsscores auf einer Skala von 0 bis 4, der den Agatston-Kalzifizierungsscore vorhersagt, unter Verwendung eines fünften trainierten neuronalen Netzes. das direkt auf die detektierten Bilder oder Teile der detektierten Bilder angewendet wird; und/oder
- Bestimmen von Plaque mit hohem Risiko für ein kardiales Ereignis unter Verwendung eines sechsten trainierten neuronalen Netzes, das direkt auf die detektierten Bilder oder Teile der detektierten Bilder angewendet wird.

13. Medium nach Anspruch 12, **dadurch gekennzeichnet, dass** es das Durchführen der folgenden fünf Vorgänge durch den Prozessor bewirkt:

- Vorhersagen eines Intervalls für den Wert des Koronarreservefluss unter Verwendung eines zweiten trainierten tiefen neuronalen Netzes, das direkt auf die detektierten Bilder oder Teile der detektierten Bildern angewendet wird;
- Bestimmen eines Wertes gemäß der CAD-RADS-Klassifizierung unter Verwendung eines dritten trainierten tiefen neuronalen Netzes, das direkt auf die detektierten Bilder oder Teile der detektierten Bilder angewendet wird;
- automatisiertes Bestimmen der Bildqualität, die einen diagnostischen Vertrauensindex bereitstellt, unter Verwendung eines vierten trainierten neuronalen Netzes, das direkt auf die detektierten Bilder oder Teile der detektierten Bildern angewendet wird;
- Bestimmen eines globalen Kalzifizierungsscores auf einer Skala von 0 bis 4, der den Agatston-Kalzifizierungsscore vorhersagt, unter Verwendung eines fünften trainierten neuronalen Netzes, das direkt auf die detektierten Bilder oder Teile der detektierten Bilder angewendet wird; und
- Bestimmen von Plaque mit hohem Risiko für ein kardiales Ereignis unter Verwendung eines sechsten trainierten neuronalen Netzes, das direkt auf die detektierten Bilder oder Teile der detektierten Bilder angewendet wird.

**Claims**

1. A computer-implemented method for determining the presence of a coronary stenosis in a patient, comprising:

- a step of receiving multiplanar curved or stretched medical computed tomography (CT) images including the coronary stenosis of said patient, said images including representations of the coronary stenosis viewed under different angles;
- a step of detecting said coronary stenosis in said images or in a portion of said images by using a first trained deep neural network configured to read said images;
- a step of predicting a value interval of fractional flow reserve (FFR) by using a second trained deep neural network applied directly to said detected images or detected image portions.

2. Method according to claim 1, **characterized in that** it further comprises a step of determining a value according to the CAD-RADS (Coronary Artery Disease - Reporting and Data System) classification of a coronary stenosis by using a third trained neural network applied directly to the detected images or image portions.

3. Method according to claim 1 or 2, **characterized in that** it further comprises at least one of the following steps, which may be carried out in any order:

- a step of automated image quality determination providing a diagnostic confidence index by using a fourth trained neural network applied directly to the detected images or image portions;
- a step of determining a global calcification score on a scale from 0 to 4 predicting the Agatston calcium score category by using a fifth trained neural network applied directly to the detected images or image portions; and/or
- a step of determining high-risk plaque (HRP) of cardiac event by using a sixth trained neural network applied directly to the detected images or image portions.

4. Method according to claim 3, **characterized in that** it further comprises a step of predicting a value interval of fractional flow reserve (FFR) by manual, semi-automated and/or automated measurement of at least two morphological criteria selected from:

• minimum diameter of the stenosis in mm;
• minimum area of the stenosis in mm$^2$;
• maximum degree of coronary stenosis expressed as a diameter percentage (%);
• maximum degree of coronary stenosis expressed as an area percentage (%);
• length of the stenosis in mm; and/or
• myocardial mass or percentage (%) of myocardial mass downstream of the coronary stenosis.

5. Method according to any one of the preceding claims, **characterized in that** the images or image portions are derived from a coronary CT angiography (CCTA).

6. A device suitable for determining the presence of a coronary stenosis in a patient, comprising:

- means for receiving multiplanar curved medical computed tomography (CT) images including the coronary stenosis of said patient, said images including representations of the coronary stenosis viewed under different angles;
- means for detecting said coronary stenosis in said images or in a portion of said images by using a first trained deep neural network configured to read said images;

**characterized in that** it further comprises means for predicting a value interval of fractional flow reserve by using a second trained deep neural network applied directly to said detected images or detected image portions.

7. Device according to claim 6, **characterized in that** it further comprises means for determining a value according to the CAD-RADS classification by using a third trained deep neural network applied directly to the detected images or image portions.

8. Device according to claim 6 or 7, **characterized in that** it further comprises at least one of the following means:

- means for automated image quality determination providing a diagnostic confidence index by using a fourth trained neural network applied directly to the detected images or image portions;
- means for determining a global calcification score on a scale from 0 to 4 predicting the Agatston calcium score by using a fifth trained neural network applied directly to the detected images or image portions; and/or
- means for determining high-risk plaque of cardiac event by using a sixth trained neural network applied directly to the detected images or image portions.

9. Device according to claim 8, **characterized in that** it comprises the following five means:

- means for predicting a value interval of fractional flow reserve by using a second trained deep neural network applied directly to the detected images or image portions;
- means for determining a value according to the CAD-RADS classification by using a third trained deep neural network applied directly to the detected images or image portions;
- means for automated image quality determination providing a diagnostic confidence index by using a fourth trained neural network applied directly to the detected images or image portions;
- means for determining a global calcification score on a scale from 0 to 4 predicting the Agatston calcium score by using a fifth trained neural network applied directly to the detected images or image portions; and
- means for determining high-risk plaque of cardiac event by using a sixth trained neural network applied directly to the detected images or image portions.

10. A non-transitory computer-readable medium storing computer-readable program instructions for determining the presence of a coronary stenosis in a patient, comprising execution by a processor of computer-readable program instructions configured to carry out the following operations:

- receiving multiplanar curved medical computed tomography (CT) images including the coronary stenosis of said patient, said images including representations of the coronary stenosis viewed under different angles;
- detecting said potentially hemodynamically significant coronary stenosis in said images or in a portion of said images by using a first trained deep neural network configured to read said images;

**characterized in that** it further causes the processor to perform a prediction operation of a value interval of fractional flow reserve by using a second trained deep neural network applied directly to said detected images or detected image portions.

11. Medium according to claim 10, **characterized in that** it further causes the processor to perform an operation of determining a value according to the CAD-RADS classification by using a third trained deep neural network applied directly to the detected images or image portions.

12. Medium according to claim 10 or 11, **characterized in that** it further causes the processor to perform at least one of the following operations:

- automated image quality determination providing a diagnostic confidence index by using a fourth trained neural network applied directly to the detected images or image portions;
- determination of a global calcification score on a scale from 0 to 4 predicting the Agatston calcium score by using a fifth trained neural network applied directly to the detected images or image portions; and/or
- determination of high-risk plaque of cardiac event by using a sixth trained neural network applied directly to the detected images or image portions.

13. Medium according to claim 12, **characterized in that** it causes the processor to perform the following five operations:

- prediction of a value interval of fractional flow reserve by using a second trained deep neural network applied directly to the detected images or image portions;
- determination of a value according to the CAD-RADS classification by using a third trained deep neural network applied directly to the detected images or image portions;
- automated image quality determination providing a diagnostic confidence index by using a fourth trained neural network applied directly to the detected images or image portions;
- determination of a global calcification score on a scale from 0 to 4 predicting the Agatston calcium score by using a fifth trained neural network applied directly to the detected images or image portions; and
- determination of high-risk plaque of cardiac event by using a sixth trained neural network applied directly to the detected images or image portions.

[Fig. 1]

[Fig. 2]

[Fig. 3]

[Fig. 4]

[Fig. 5]

| Concept prédit | Confiance |
|---|---|
| FFR+ | 99.94% |
| FFR- | 0.06% |

[Fig. 6]

**Métriques au seuil optimal (0,35) pour la catégorie FFR+:**

[Fig. 7]

Résultats

Fréquence

Classification

FFR+

[Fig. 8]

[Fig. 9]

Résultats:
FFR- moyenne: 0,497
FFR+ moyenne: 0,503

Image 0

Recadrage

Résultats:
FFR- : 0,562
FFR+ : 0,436

Image 1

Recadrage

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20150112182 A

**Littérature non-brevet citée dans la description**

- **KERL et al.** 64-Slice Multidetector-row Computed Tomography in the Diagnosis of Coronary Artery Disease: Interobserver Agreement Among Radiologists With Varied Levels of Experience on a Per-patient and Per-segment Basis.. *J Thorac Imaging. janv*, 2012, vol. 27 (1), 29-35
- Coronary CT Angiography and 5-Year Risk of Myocardial Infarction. *N Engl J Med.*, 06 September 2018
- **ZREIK M et al.** A Recurrent CNN for Automatic Detection and Classification of Coronary Artery Plaque and Stenosis in Coronary CT Angiography. *IEEE Trans Med Imaging.*, 2018
- **TONINO et al.** Fractional Flow Reserve versus Angiography for Guiding Percutaneous Coronary Intervention. *N Engl J Med.*, 15 January 2009
- **TONINO et al.** Angiographic Versus Functional Severity of Coronary Artery Stenoses in the FAME Study. *J Am Coll Cardiol.*, June 2010
- **TESCHE et al.** Coronary CT angiography derived morphological and functional quantitative plaque markers correlated with invasive fractional flow reserve for detecting hemodynamically significant stenosis. *J Cardiovasc Comput Tomogr.*, May 2016
- **NAKANISHI et al.** Automated estimation of image quality for coronary computed tomographic angiography using machine learning.. *Eur Radiol.*, September 2018
- **LOSSAU et al.** Motion artifact recognition and quantification in coronary CT angiography using convolutional neural networks. *Med Image Anal.*, February 2019